# EUROPÄISCHE PATENTSCHRIFT

(11) **EP 1 369 406 B1**
(45) Veröffentlichungstag und Bekanntmachung des Hinweises auf die Patenterteilung: **07.06.2006**
(21) Anmeldenummer: 03010340.2
(22) Anmeldetag: 08.05.2003
(51) Int. Cl.: C07C 25/22, C07C 43/225, C09K 19/32, G02F 1/13

(54) **Fluorierte Naphthaline, diese enthaltende Flüssigkristallmischungen und Flüssigkristallanzeigen**
Fluorinated naphthalene derivatives, liquid crystal compositions and liquid crystal displays containing them
Naphtalènes fluorinés, compositions liquides cristallines et dispositifs à cristaux liquides les contenant

(30) Priorität: 07.06.2002 DE 10225427
(43) Veröffentlichungstag der Anmeldung: 10.12.2003
(73) Patentinhaber: Merck Patent GmbH, 64293 Darmstadt (DE)
(72) Erfinder: Bremer, Matthias, Dr., 64295 Darmstadt (DE); Klasen-Memmer, Melanie, Dr., 67259 Heuchelheim (DE); Hornung, Barbara, 63594 Hasselroth (DE); Schmidt, Wolfgang, 63303 Dreieich (DE); Wingen, Rainer, 65795 Hattersheim (DE)

(56) Entgegenhaltungen:
- EP-A- 0 826 659
- WO-A-98/27179
- DE-A- 19 522 152
- DE-A- 19 748 438

## Beschreibung

Für die Verwendung in flüssigkristallinen Mischungen sind fluorierte Derivate des Naphthalins z.B. aus US 6159392, US 6159561 oder Mol. Cryst. Liq. Cryst. 364, 865 (2001) bekannt.

In EP 0 826 659 ist eine generelle Formel von Verbindungen mit fluorierten Dihydronaphthylringen bekannt. Dieses Dokument beschreibt jedoch weder konkrete Verbindungen mit mehr als einem Fluoratom im Dihydronaphthalin noch Verbindungen, bei denen der aromatische Ring fluoriert ist. Insbesondere die letzteren zeichnen sich jedoch durch besonders günstige dielektrische Eigenschaften aus.

Da aber die Entwicklung von Flüssigkristallmischungen noch in keiner Weise als abgeschlossen betrachtet werden kann, sind die Hersteller von Displays an den unterschiedlichsten Komponenten für Mischungen interessiert.

Insbesondere werden Flüssigkristallmischungen benötigt, die einerseits einen sehr weiten Arbeits-Temperaturbereich aufweisen, andererseits eine möglichst geringe Schwellspannung besitzen, beispielsweise für den Einsatz in Automobilen, in denen ohne weiteres ein Temperaturbereich von -40°C bis 100°C auftreten kann, aber auch für tragbare Geräte wie Mobiltelefone und Notebook-PCs.

Es besteht daher eine anhaltende Nachfrage nach neuen, geeigneten Flüssigkristallmischungen und -Mischungskomponenten.

Aufgabe der vorliegenden Erfindung ist es daher, neue Komponenten für die Verwendung in nematischen oder cholesterischen Flüssigkristallmischungen bereit zu stellen, die über hohe Absolutwerte der dielektrischen Anisotropie negativen Vorzeichens kombiniert mit einem günstigen Verhältnis von Viskosität und Klärpunkt verfügen. Darüber hinaus sollen die Verbindungen in hohem Maße licht- und UVstabil sowie thermisch stabil sein. Ferner sollen sie geeignet sein, eine hohe ,voltage holding ratio (VHR)' zu realisieren. Ferner sollen sie synthetisch gut zugänglich und daher potentiell kostengünstig sein.

Es wurde nun gefunden, dass diese Anforderungen erfüllt werden von den fluorierten Naphthalinen der Formel (I) 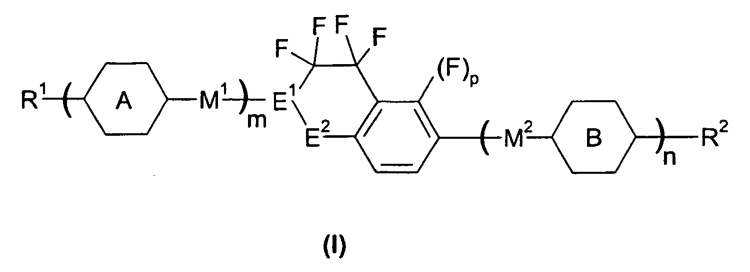 worin bedeuten:
- **R**^{**1**}**, R**^{**2**}: unabhängig voneinander H, einen linearen Alkylrest mit 1 bis 12 C-Atomen oder einen linearen oder verzweigten Alkenylrest mit 2 bis 8 C-Atomen, worin auch
a) eine (nicht terminale) -CH₂-Gruppe durch -O- oder -C(=O)O-ersetzt sein kann und /oder
b) eine -CH₂-Gruppe durch -C≡C-, Cyclopropan-1,2-diyl oder Cyclobutan-1,3-diyl ersetzt sein kann und/oder
c) mehrere H durch F ersetzt sein können
mit der Maßgabe, dass **R**^{**1**}**, R**^{**2**} nicht zugleich H bedeuten sollen.
- **M**^{**1**}**, M**^{**2**}: unabhängig voneinander -C(=O)O-, -OC(=O)- , -CH₂O-, -OCH2-C≡C- , -CH₂CH₂- , -(CH₂)₄-, -CF₂CF₂-, -CF₂O-, -OCF₂- oder eine Einfachbindung
- >**E**¹-**E**²-: die Gruppierung >C=CH- oder >CH-CH₂-
- **m, n**: unabhängig voneinander Null, 1 oder 2 mit der Maßgabe, dass die Summe m + n 0, 1 oder 2 beträgt
- **p**: Null oder 1 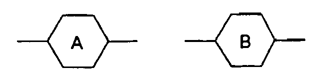
unabhängig voneinander Phenylen-1,4-diyl, gegebenenfalls ein-, zwei- oder dreifach durch F substituiert, Cyclohexan-1,4-diyl, gegebenenfalls ein- oder zweifach durch F substituiert, 1-Cyclohexen-1,4-diyl, gegebenenfalls einfach durch F substituiert oder 1,3-Dioxan-2,5-diyl

Bevorzugt sind die Verbindungen der Formel (I) bei denen >E¹-E²- die Bedeutung >C=CH- hat.

Ebenfalls bevorzugt sind die Verbindungen der Formel (I) mit der Bedeutung p=1.

Ebenfalls bevorzugt sind die Verbindungen der Formel (I) mit den Bedeutungen m = 1 oder 2; n = 0; M¹ und M² = Einfachbindung.

Ebenfalls bevorzugt sind die Verbindungen der Formel (I) mit den Bedeutungen m = 0; n = 1 oder 2; M¹ und M² = Einfachbindung.

Ebenfalls bevorzugt sind die Verbindungen der Formel (I) mit den Bedeutungen m =1 oder 2; 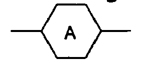 Phenylen-1,4-diyl oder Cyclohexan-1-4-diyl.

Ebenfalls bevorzugt sind die Verbindungen der Formel (I) mit den Bedeutungen
n = 1 oder 2, 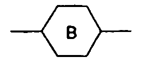 Phenylen-1,4-diyl oder Cyclohexan-1,4-diyl..

Besonders bevorzugt sind die Verbindungen der Formel (I) mit den Bedeutungen
E¹-E² = >C=CH-; p = 1; m = 1 oder 2; n = 0; 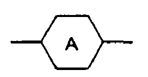 Phenylen-1,4-diyl oder Cyclohexan-1,4-diyl.
Besonders bevorzugt sind auch die Verbindungen der Formel (I) mit den Bedeutungen
>E¹-E²- = >C=CH-; p = 1; m = 0; n = 1oder 2; 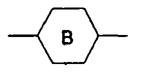 Phenylen-1 ,4-diyl oder Cyclohexan-1,4-diyl.

Speziell bevorzugt sind die Verbindungen der Formeln (la) bis (Ij): 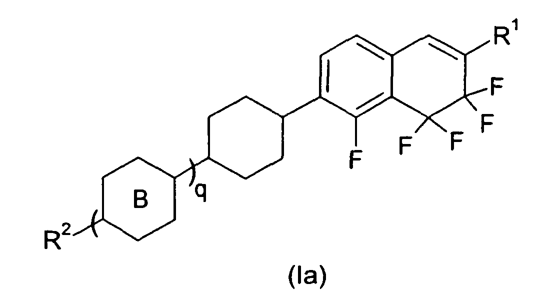 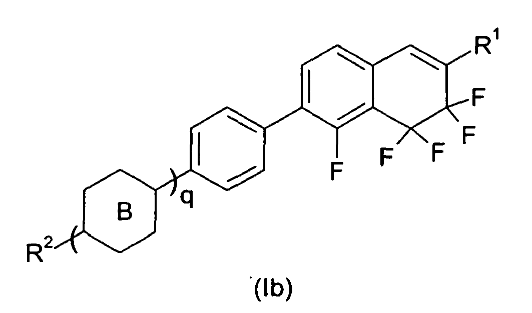 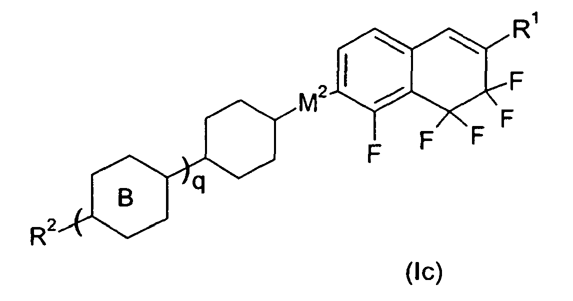 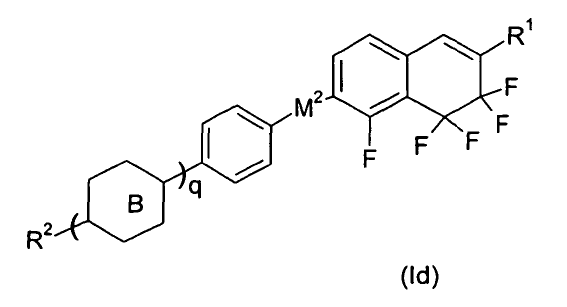 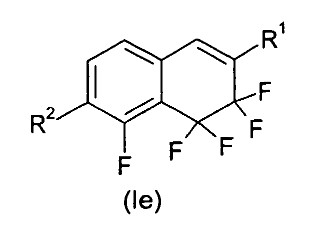 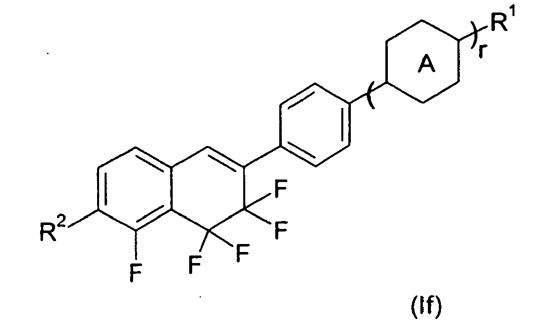 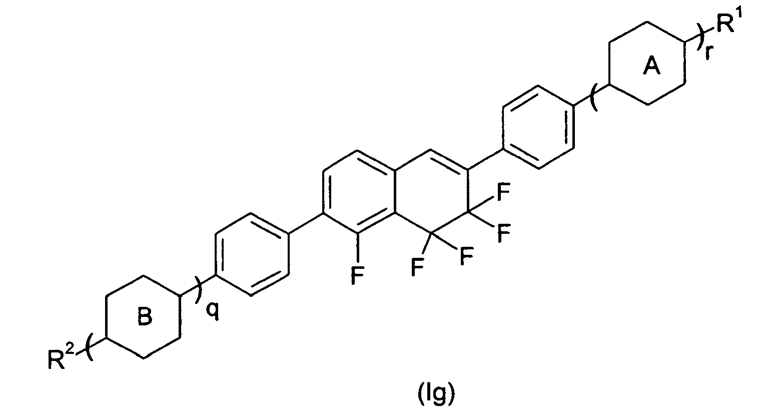 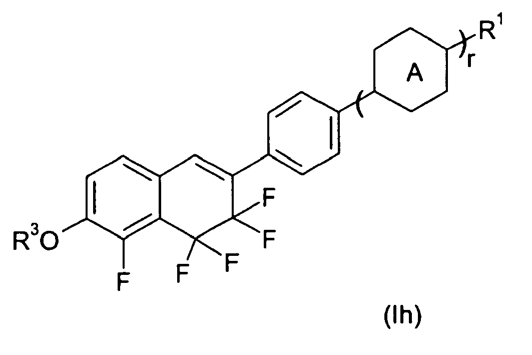 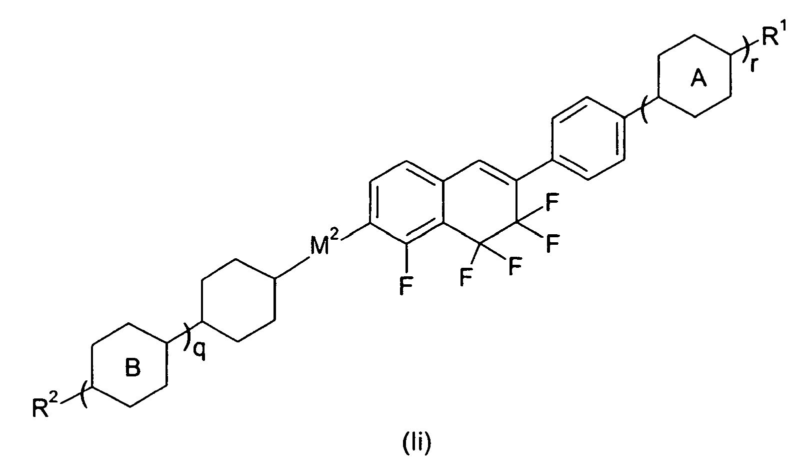 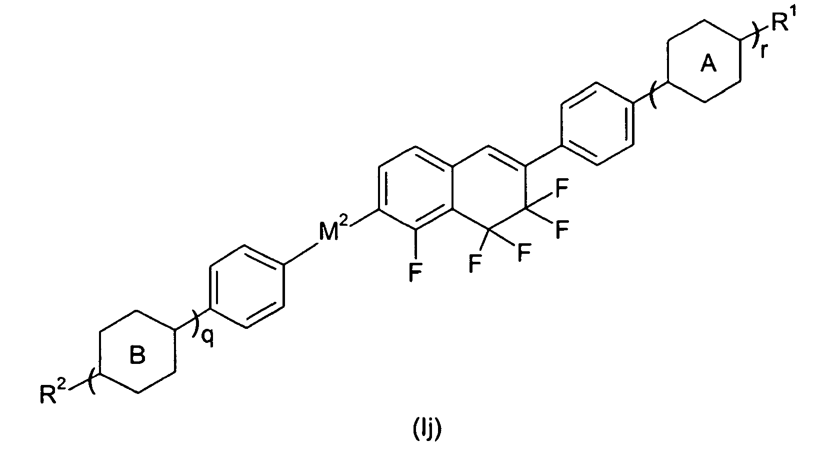
- R³: bedeutet einen Alkylrest mit 1 bis 12 C-Atomen oder einen Alkenylrest mit 2 bis 8 C-Atomen.
- q: bedeutet 0 oder 1.
- r: bedeutet 0 oder 1 mit der Maßgabe, dass die Summe q + r in den Verbindungen
der Formeln (lg), (li) und (lj) 0 oder 1 ist.

Ganz speziell bevorzugt sind die Verbindungen der Formeln 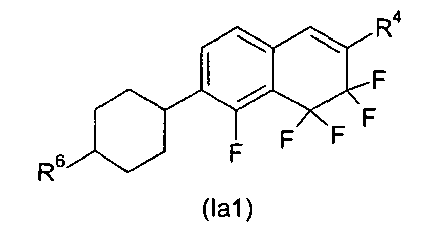 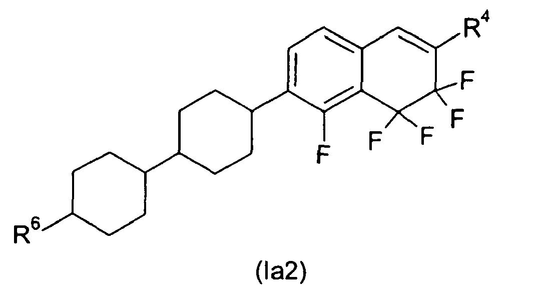 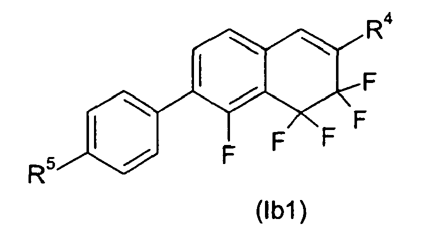 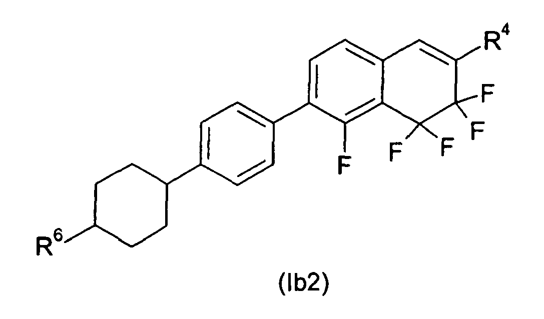 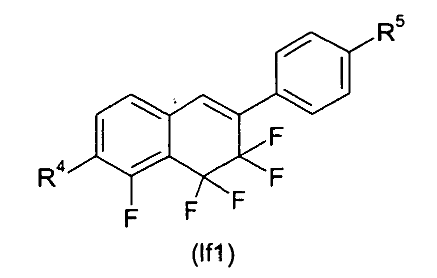 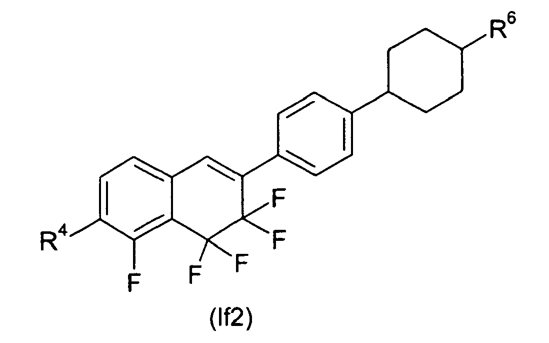 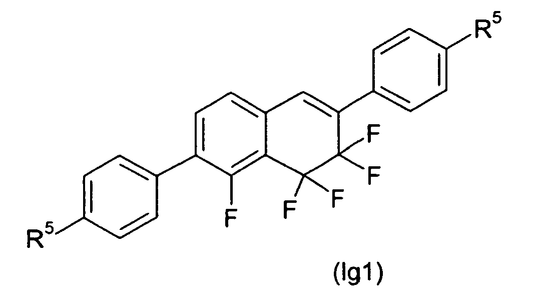 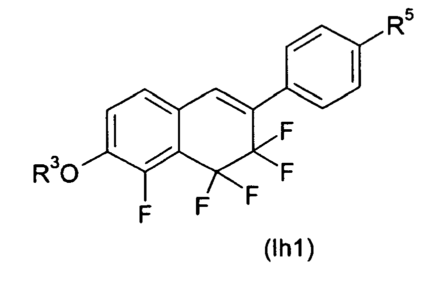 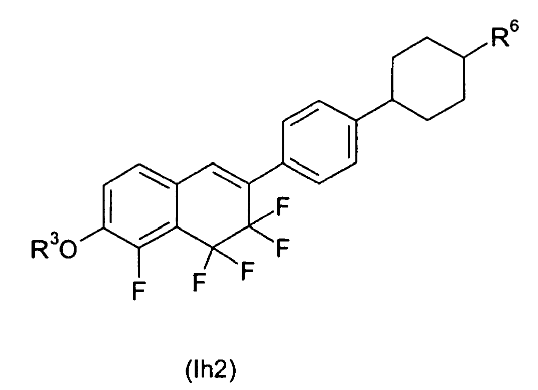
- R⁴: bedeutet einen Alkylrest mit 1 bis 6 C-Atomen oder einen Alkenylrest mit 2 bis 6 C-Atomen, worin auch jeweils eine (nicht endständige) -CH₂-Gruppe durch -O- ersetzt sein kann
- R⁵: bedeutet einen Alkylrest mit 1 bis 6 C-Atomen oder einen Alkenylrest mit 2 bis 6 C-Atomen, worin auch jeweils eine (nicht endständige) -CH₂₋Gruppe durch -O- ersetzt sein kann
- R⁶: bedeutet einen Alkylrest mit 1 bis 6 C-Atomen

Mit der Bereitstellung von Verbindungen der Formel (I) wird ganz allgemein die Palette der flüssigkristallinen Substanzen, die sich unter verschiedenen anwendungstechnischen Gesichtspunkten zur Herstellung flüssigkristalliner Gemische eignen, erheblich verbreitert.

In diesem Zusammenhang besitzen die Verbindungen der Formel (I) einen breiten Anwendungsbereich. In Abhängigkeit von der Auswahl der Substituenten können sie anderen Verbindungsklassen zugesetzt werden, um beispielsweise die dielektrische und/oder optische Anisotropie eines solchen Dielektrikums zu beeinflussen. Auch können sie dazu dienen, um dessen Schwellenspannung und/oder dessen Viskosität zu optimieren.

Besonders geeignet sind die Verbindungen der Formel (I), um schon in geringen Zumischmengen die dielektrische Anisotropie (Δε) zu beeinflussen, insbesondere höhere Absolutwerte der dielektrischen Anisotropie negativen Vorzeichens zu erzielen.

Gegenstand der Erfindung sind somit Verbindungen der Formel (I) sowie die Verwendung dieser Verbindungen als Komponenten flüssigkristalliner Mischungen und Flüssigkristallmischungen enthaltend Verbindungen der Formel (I).

Die Verbindungen der Formel (I) werden vorzugsweise in nematischen oder cholesterischen Flüssigkristallmischungen eingesetzt. Die erfindungsgemäßen Flüssigkristallmischungen enthalten mindestens eine Verbindung der Formel (I), vorzugsweise in einer Menge von 1 bis 80 Gew.-% besonders von 3 bis 40 Gew.-%, bezogen auf die Flüssigkristallmischung. Vorzugsweise enthalten sie mindestens 3 weitere Verbindungen. Die Auswahl dieser weiteren Verbindungen (z.B. aus den in DE-A-196 29 812, S.12 bis 16 aufgelisteten Typen) sowie die Herstellung der Flüssigkristallmischungen ist dem Fachmann geläufig.

Die Erfindung betrifft auch ein Flüssigkristalldisplay, das diese Flüssigkristallmischungen enthält. Bevorzugt arbeitet dieses Flüssigkristalldisplay im IPS-Anzeigemodus (Kiefer et al., Japan Display '92, S.547) oder im VA-Anzeigemodus (Ohmura et al., SID 97 Digest, S.845) oder im ECB Anzeigemodus (EP-A-0 474 062).

Ebenfalls bevorzugt werden die Verbindungen der Formel (I) in chiralsmektischen Flüssigkristallmischungen eingesetzt. Die erfindungsgemäßen Flüssigkristallmischungen enthalten mindestens eine Verbindung der Formel (I), vorzugsweise in einer Menge von 1 bis 40 Gew.-%, bezogen auf die Flüssigkristallmischung. Vorzugsweise enthalten sie mindestens 3 weitere Komponenten. Vorzugsweise werden diese Komponenten ausgewählt aus den bekannten Verbindungen mit smektischen und/ oder nematischen und/oder cholesterischen Phasen. Die Auswahl dieser weiteren Verbindungen (z.B. aus den in DE-A-198 57 352 aufgelisteten Typen) sowie die Herstellung der Flüssigkristallmischungen ist dem Fachmann geläufig.

Die Erfindung betrifft auch ein Flüssigkristalldisplay, das diese Flüssigkristallmischungen enthält.

Die erfindungsgemäßen Anzeigeelemente (Displays) sind üblicherweise so aufgebaut, dass eine Flüssigkristallschicht beiderseitig von Schichten eingeschlossen ist, die üblicherweise, in dieser Reihenfolge ausgehend von der LC-Schicht, mindestens eine Orientierungsschicht, Elektroden und eine Begrenzungsscheibe (z.B. aus Glas) sind. Darüber hinaus können sie Abstandshalter, Kleberahmen, Polarisatoren sowie für Farbdisplays dünne Farbfilterschichten enthalten. Weitere mögliche Komponenten sind Antireflex-, Passivierungs-, Ausgleichs- und Sperrschichten sowie elektrisch-nichtlineare Elemente, wie Dünnschichttransistoren (TFT) und Metall-Isolator-Metall-(MIM)-Elemente. Im Detail ist der Aufbau von Flüssigkristalldisplays bereits in einschlägigen Monographien beschrieben (siehe z.B. E. Kaneko, "Liquid Crystal TV Displays: Principles and Applications of Liquid Crystal Displays", KTK Scientific Publishers, 1987).

Besonders bevorzugt werden die erfindungsgemäßen Verbindungen in Anzeigen vom AMD-TN-, AMD-IPS- oder AMD-VA (auch AMD-ECB)-Typ verwendet.

Beispielhaft sind in den nachfolgenden Schemata mögliche Synthesewege zu Verbindungen der Formel (I) angegeben, wobei auch andere Verfahren denkbar und möglich sind.

### Folgende Abkürzungen werden verwendet:

- n-BuLi: n-Buthyllithium
- DAST: Diethylamino-schwefeltrifluorid
- DCC: Dicyclohexylcarbodiimid
- DDQ: 2,3-Dichlor-5,6-dicyan-p-benzochinon
- DEAD: Diethylazodicarboxylat (Azodicarbonsäurediethylester)
- Diglyme: Diethylenglykoldimethylether
- DMAP: 4-(Dimethylamino)pyridin
- DME: Dimethoxyethan
- DMF: N,N-Dimethylformamid
- F-TEDA-BF₄: 1-Chlormethyl-4-fluor-1,4-diazoniabicyclo[2.2.2]octan bis-(tetrafluoroborat)
- HOAc: Essigsäure
- KOtBu: Kalium-tert-butylat
- LICOR: Lithiumorganyl + Kalium-tert-butylat
- LiTMP: Lithium-2,2,6,6-tetramethylpiperidid
- MTBE: tert-Butylmethylether
- NMP: N-Methylpyrrolidon
- (Tf)₂O: Trifluormethansulfonsäureanhydrid
- 4-TsOH: 4-Toluolsulfonsäure

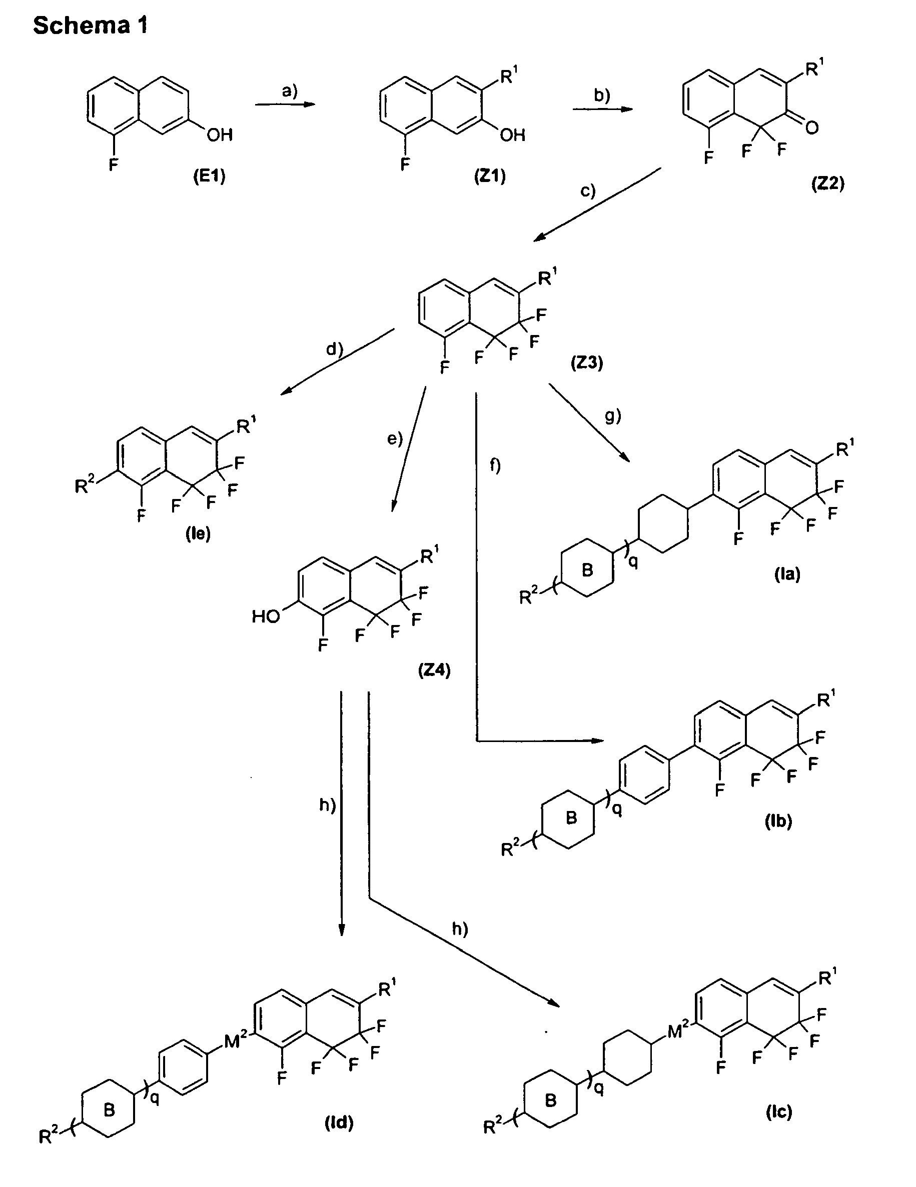

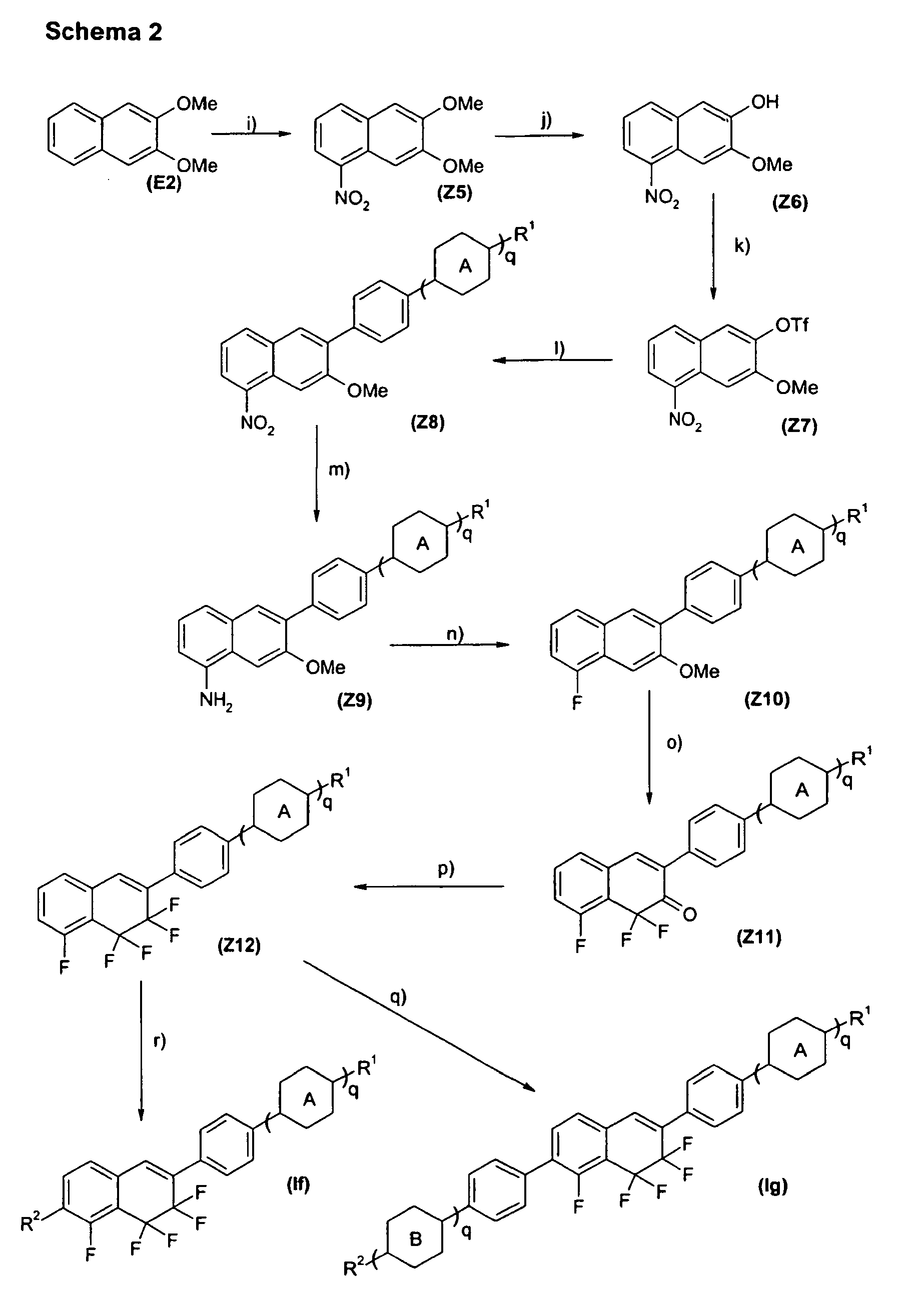

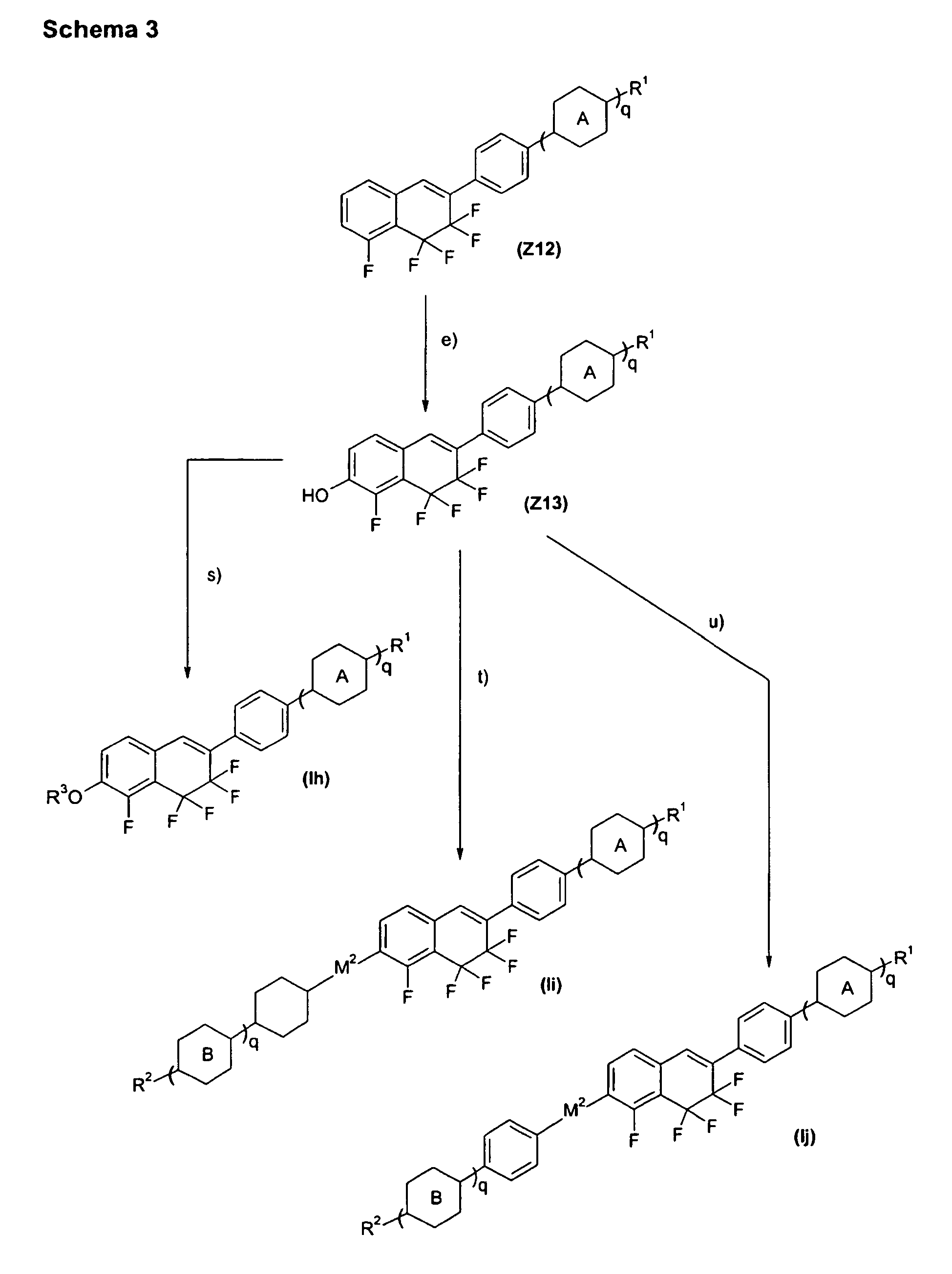

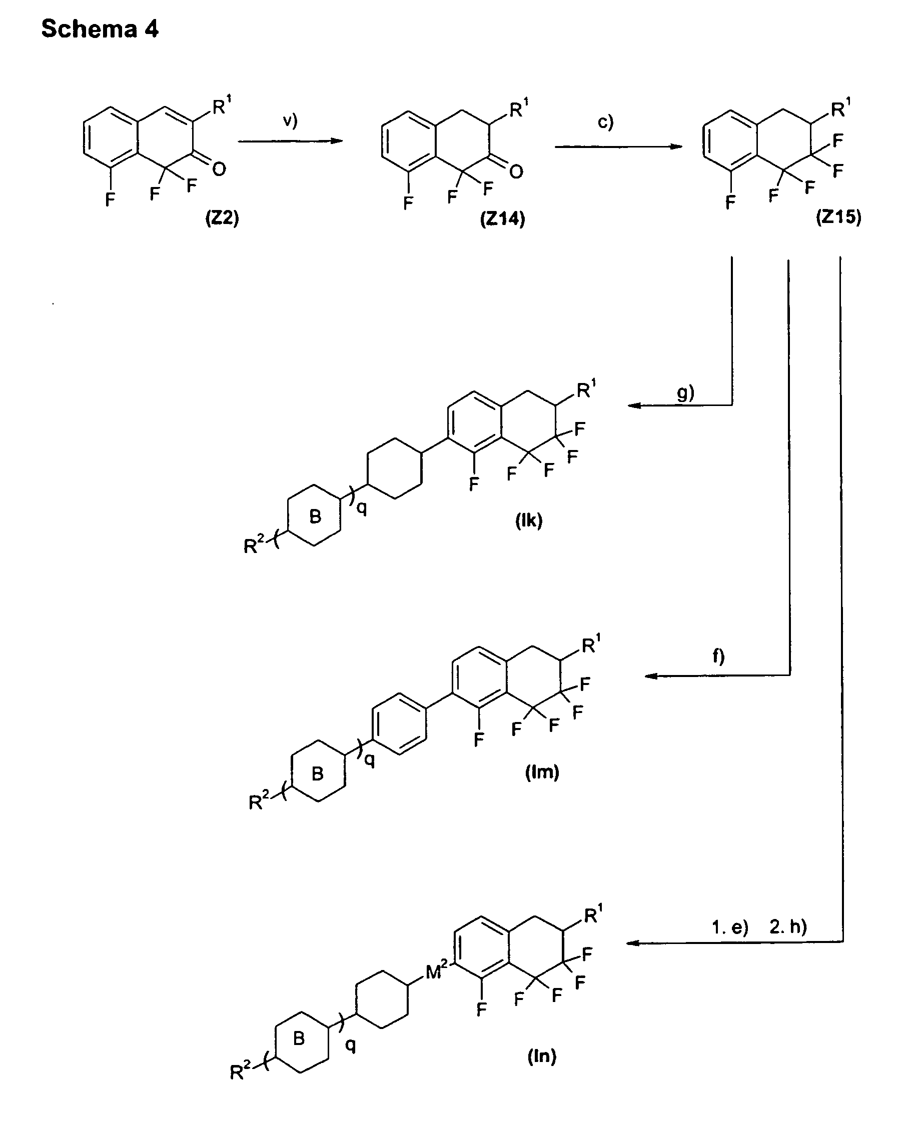

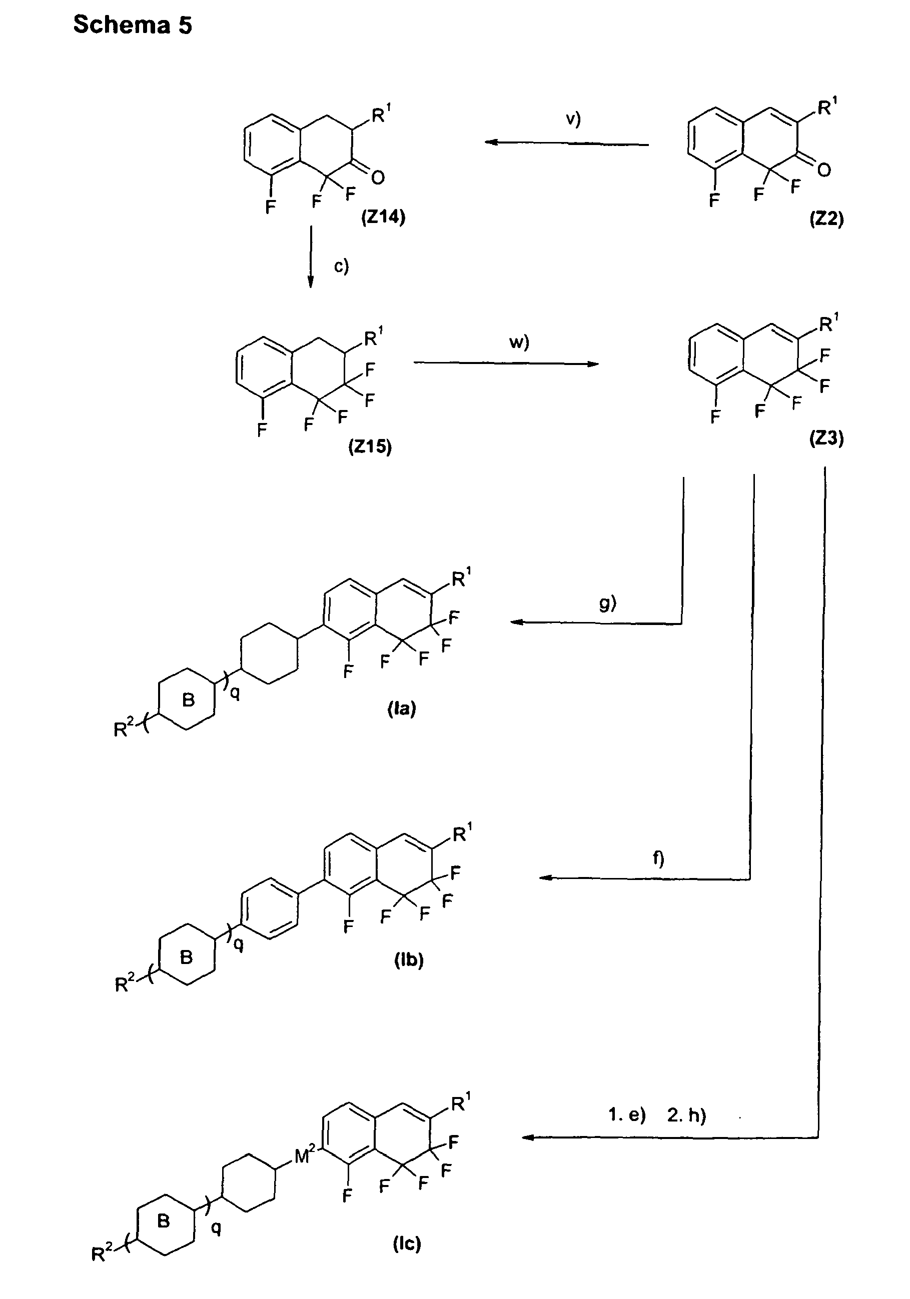

a) C₂H₄ (R¹ = C₂H₅), AlCl₃, CH₂Cl₂, Bestrahlung *analog Kakiuchi, J. Org. Chem. 58,* 2797 (1993)
b) F-TEDA-BF₄, CH₃CN *analog EP-A 0 952135*
c) DAST, CH₂Cl₂ *analog EP-A 0 952135*
d) 1.ortho-Lithiierung *analog J.Chem.Soc.Perkin Trans I 1995, 2729* 2. R²-X *analog Recl. Trav. Chim. Pays-Bas 113, 529 (1994)*
e) 1.ortho-Lithiierung 2. B(OMe)₃ 3.H⁺ 4. H₂O₂/ Et₂O *analog J. Chem. Soc. Perkin Trans* //*1989, 2041*
f) 1.ortho-Lithiierung 2. B(OMe)₃ 3.H⁺ 4. R²-(B)_{q}-C₆H₄-Br, Pd-Katalysator, Na₂CO₃, Toluol/Ethanol/H₂O *analog J. Chem. Soc. Perkin Trans II 1989*, *2041*
g) 1.ortho-Lithiierung 2. R²-(B)_{q}-cyclohexanon 3. H⁺ 4. H₂, Pd-C (5%), Toluol *analog WO 96*/*00710*
h) z.B. R²-(B)_{q}-C₆H₁₀CH₂Br, K₂CO₃, Butanon-2 für M² = -CH₂O- oder R²⁻(B)_{q}-C₆H₄CO₂H, DCC, CH₂Cl₂ für M² = -C(=O)O-
i) HNO₃/HOAc : *Gillespie, J.Org.Chem. 39, 3239 (1974)*
j) HBr/HOAc: *Gillespie, J.Org.Chem. 39, 3239 (1974)*
k) Trifluormethansulfonsäureanhydrid / Pyridin *analog Liq. Cryst. 18, 1 (1995)*
l) R²-(A)_{q}-C₆H₄-Br, Pd-Katalysator, Na₂CO₃, Toluol/Ethanol/H₂O *analog J. Chem. Soc. Perkin Trans II 1989*, *2041*
m) Sn, HCI *analog Dewar, J.Am.Chem.Soc. 84, 3782 (1962)*
n) 1. NaNO₂ 2. HBF₄ 3.Thermolyse *analog Corral, Heterocycles 23, 1431 (1985)*
o) F-TEDA-BF₄, CH₃CN *analog EP-A 0 952135*
p) DAST, CH₂Cl₂ *analog EP-A 0 952135*
q) 1.ortho-Lithiierung 2. B(OMe)₃ 3.H⁺ 4. R²-(B)_{q}-C₆H₄-Br, Pd-Katalysator, Na₂CO₃, Toluol/Ethanol/H₂O *analog J. Chem. Soc. Perkin Trans II 1989*, *2041*
r) 1.ortho-Lithiierung *analog J.Chem.Soc.Perkin Trans I 1995, 2729 2*. R²-X *analog Recl. Trav. Chim. Pays-Bas 113, 529 (1994)*
s) R³-X, K₂CO₃, Propanon-2 (X = Br, I)
t) z.B. R²-(B)_{q}-C₆H₁₀CH₂Br, K₂CO₃, Butanon-2 für M² = -CH₂O-
u) z.B. R²-(B)_{q}-C₆H₄CO₂H, DCC, CH₂Cl₂ für M² = -C(=O)O-
v) H₂, Pd /C *analog Tetrahedron Letters 40, 3827 (1999)*
w) DDQ, Toluol *analog EP-B 946474*

Die Ausgangsmaterialien sind literaturbekannt:
(E1): [13916-98-8]: Adcock, J. Am. Chem. Soc. 89, 386 (1967)
(E2): [10103-06-7], (Z6): [7311-22-0]: Bell, J. Chem. Soc. C, 904 (1966)

Die weitere Umsetzung der funktionellen Derivate der Verbindungen (I) in den Schemata 1 bis 5 zu den Endverbindungen erfolgt nach dem Fachmann geläufigen Methoden.

Bevorzugt werden in den Flüssigkristallmedien gemäß der vorliegenden Anmeldung Verbindungen der Formeln I und II in einer Konzentration bis zu ca. 25 % je Einzelsubstanz und Verbindungen der Formel III in einer Konzentration bis zu ca. 20 %, bevorzugt bis zu 16 %, je Einzelsubstanz eingesetzt. Verbindungen der Formel I, bevorzugt der Formeln I-1 bis I-3, werden bevorzugt in Konzentrationen bis zu ca. 15 %, bevorzugt bis zu 10 %, je Einzelsubstanz eingesetzt.

In der vorliegenden Anmeldung bedeutet "≤" kleiner oder gleich, bevorzugt kleiner und "≥" größer oder gleich, bevorzugt größer.

In der vorliegenden Anmeldung bedeuten 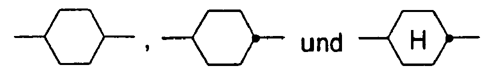 trans-1,4-cyclohexylen.

In der vorliegenden Anmeldung bedeuten die Begriffe dielektrisch positive Verbindungen solche Verbindungen mit einem Δε > 1,5, dielektrisch neutrale Verbindungen solche mit -1,5 ≤ Δε≤ 1,5 und dielektrisch negative Verbindungen solche mit Δε < -1,5. Hierbei wird die dielektrische Anisotropie der Verbindungen bestimmt, indem 10 % der Verbindungen in einem flüssigkristallinen Host gelöst werden und von dieser Mischung die Kapazität in mindestens jeweils einer Testzelle mit 20 µm Schichtdicke mit homeotroper und mit homogener Oberflächenorientierung bei 1 kHz bestimmt wird. Die Meßspannung beträgt typischerweise 0,5 V bis 1,0 V, jedoch stets weniger als die kapazitive Schwelle der jeweiligen Flüssigkristallmischung.

Als Hostmischung wird generell ZLI-4792 jedoch für die Bestimmung der dielektrischen Anisotropie von dielektrisch negativen Verbindungen ZLI-2857, beide von Merck KGaA, Deutschland, verwendet. Aus der Änderung der Dielektrizitätskonstanten der Hostmischung nach Zugabe der zu untersuchenden Verbindung und Extrapolation auf 100 % der eingesetzten Verbindung werden die Werte für die jeweiligen zu untersuchenden Verbindungen erhalten.

Der Begriff Schwellenspannung bezieht sich üblicher Weise auf die optische Schwelle für 10 % relativen Kontrast (V₁₀), sofern nicht explizit anders angegeben.

In der vorliegenden Anmeldung wird jedoch in Bezug auf die Flüssigkristallmischungen mit negativer dielektrischer Anisotropie der Begriff Schwellenspannung für die kapazitive Schwellenspannung (V₀) auch Freedericksz-Schwelle genannt, verwendet, sofern nicht explizit anders angegeben.

Alle Konzentrationen in dieser Anmeldung, soweit nicht explizit anders vermerkt, sind in Massenprozent angegeben und beziehen sich auf die entsprechende Mischung oder Mischungskomponente. Alle physikalischen Eigenschaften werden und wurden nach "Merck Liquid Crystals, Physical Properties of Liquid Crystals", Status Nov. 1997, Merck KGaA, Deutschland bestimmt und gelten für eine Temperatur von 20 °C, sofern nicht explizit anders angegeben. Δn wird bei 589 nm und Δε bei 1 kHz bestimmt.

Bei den Flüssigkristallmedien mit negativer dielektrischer Anisotropie wurde die Schwellenspannung als kapazitive Schwelle V₀ (auch Freedericksz-Schwelle genannt) in bei Merck KGaA, Deutschland, hergestellten Testzellen mit durch die Orientierungsschicht SE 1211 der Fa. Nissan Chemicals homeotrop orientiertem Flüssigkristall bestimmt.

Die erfindungsgemäßen Flüssigkristallmedien können bei Bedarf auch weitere Zusatzstoffe und chirale Dotierstoffe in den üblichen Mengen enthalten. Die eingesetzte Menge dieser Zusatzstoffe beträgt insgesamt 0 % bis 10 % bezogen auf die Menge der gesamten Mischung bevorzugt 0,1 % bis 6 %. Die Konzentrationen der einzelnen eingesetzten Verbindungen beträgt bevorzugt 0,1 % bis 3 %. Die Konzentration dieser und ähnlicher Zusatzstoffe wird bei der Angabe der Konzentrationen sowie der Konzentrationsbereiche der Flüssigkristallverbindungen in den Flüssigkristallmedien nicht berücksichtigt.

Die Zusammensetzungen bestehen aus mehreren Verbindungen, bevorzugt aus 3 bis 30, besonders bevorzugt aus 6 bis 20 und ganz besonders bevorzugt aus 10 bis 16 Verbindungen, die auf herkömmliche Weise gemischt werden. In der Regel wird die gewünschte Menge der in geringerer Menge verwendeten Komponenten in den den Hauptbestandteil ausmachenden Komponenten gelöst, zweckmäßigerweise bei erhöhter Temperatur. Liegt die gewählte Temperatur über dem Klärpunkt des Hauptbestandteils, so ist die Vervollständigung des Lösungsvorgangs besonders leicht zu beobachten. Es ist jedoch auch möglich, die Flüssigkristallmischungen auf anderen üblichen Wegen, z.B. unter Verwendung von Vormischungen oder aus einem sogenannten "Multi Bottle System" herzustellen.

Mittels geeigneter Zusatzstoffe können die erfindungsgemäßen Flüssigkristallphasen derart modifiziert werden, dass sie in jeder bisher bekannt gewordenen Art von ECB-, VAN-, IPS-, GH- oder ASM-PA LCD-Anzeige einsetzbar sind.

Die nachstehenden Beispiele dienen zur Veranschaulichung der Erfindung, ohne sie zu beschränken. In den Beispielen sind der Schmelzpunkt T (C,N), der Übergang von der smektischen (S) zur nematischen (N) Phase T(S,N) und Klärpunkt T (N,I) einer Flüssigkristallsubstanz in Grad Celsius angegeben.

Soweit nicht anders gekennzeichnet, sind vor- und nachstehend alle Prozentzahlen Gewichtsprozente und die physikalischen Eigenschaften sind die Werte bei 20 °C, sofern nicht explizit anders angegeben.

Alle angegebenen Werte für Temperaturen in dieser Anmeldung sind °C und alle Temperaturdifferenzen entsprechend Differenzgrad, sofern nicht explizit anders angegeben.

In der vorliegenen Anmeldung und in den folgenden Beispielen sind die Strukturen der Flüssigkristallverbindungen durch Acronyme angegeben, wobei die Transformation in chemische Formeln gemäß folgender Tabellen A und B erfolgt. Alle Reste CₙH₂ₙ₊₁ und CₘH₂ₘ₊₁ sind geradkettige Alkylreste mit n bzw. m C-Atomen. Die Codierung gemäß Tabelle B versteht sich von selbst. In Tabelle A ist nur das Acronym für den Grundkörper angegeben. Im Einzelfall folgt getrennt vom Acronym für den Grundkörper mit einem Strich ein Code für die Substituenten R¹ , R², L¹, L² und L³:

| Code für R¹, | R¹ | R² | L¹ | L² | L³ |
|---|---|---|---|---|---|
| R², L¹, L², L³ | | | | | |
| nm | CₙH₂ₙ₊₁ | CₘH₂ₘ₊₁ | H | H | H |
| nOm | CₙH₂ₙ₊₁ | OCₘH₂ₘ₊₁ | H | H | H |
| nO.m | OCₙH₂ₙ₊₁ | CₘH₂ₘ₊₁ | H | H | H |
| nmFF | CₙH₂ₙ₊₁ | CₘH₂ₘ₊₁ | H | F | F |
| nOmFF | CₙH₂ₙ₊₁ | OCₘH₂ₘ₊₁ | H | F | F |
| n | CₙH₂ₙ₊₁ | CN | H | H | H |
| nN.F | CₙH₂ₙ₊₁ | CN | F | H | H |
| nN.F.F | CₙH₂ₙ₊₁ | CN | F | F | H |
| nF | CₙH₂ₙ₊₁ | F | H | H | H |
| nF.F | CₙH₂ₙ₊₁ | F | F | H | H |
| nF.F.F | CₙH₂ₙ₊₁ | F | F | F | H |
| nOF | OCₙH₂ₙ₊₁ | F | H | H | H |
| nCl | CₙH₂ₙ₊₁ | Cl | H | H | H |
| nCl.F | CₙH₂ₙ₊₁ | Cl | F | H | H |
| nCl.F.F | CₙH₂ₙ₊₁ | Cl | F | F | H |
| nCF₃ | CₙH₂ₙ₊₁ | CF₃ | H | H | H |
| nCF₃.F | CₙH₂ₙ₊₁ | CF₃ | F | H | H |
| nCF₃.F.F | CₙH₂ₙ₊₁ | CF₃ | F | F | H |
| nOCF₃ | CₙH₂ₙ₊₁ | OCF₃ | H | H | H |
| nOCF₃.F | CₙH₂ₙ₊₁ | OCF₃ | F | H | H |
| nOCF₃.F.F | CₙH₂ₙ₊₁ | OCF₃ | F | F | H |
| nOCF₂ | CₙH₂ₙ₊₁ | OCHF₂ | H | H | H |
| nOCF₂.F | CₙH₂ₙ₊₁ | OCHF₂ | F | H | H |
| nOCF₂.F.F | CₙH₂ₙ₊₁ | OCHF₂ | F | F | H |
| nS | CₙH₂ₙ₊₁ | NCS | H | H | H |
| nS.F | CₙH₂ₙ₊₁ | NCS | F | H | H |
| nS.F.F | CₙH₂ₙ₊₁ | NCS | F | F | H |
| rVsN | CᵣH₂ᵣ₊₁-CH=CH-CₛH₂ₛ- | CN | H | H | H |
| rEsN | CᵣH₂ᵣ₊₁-O-CₛH₂ₛ- | CN | H | H | H |

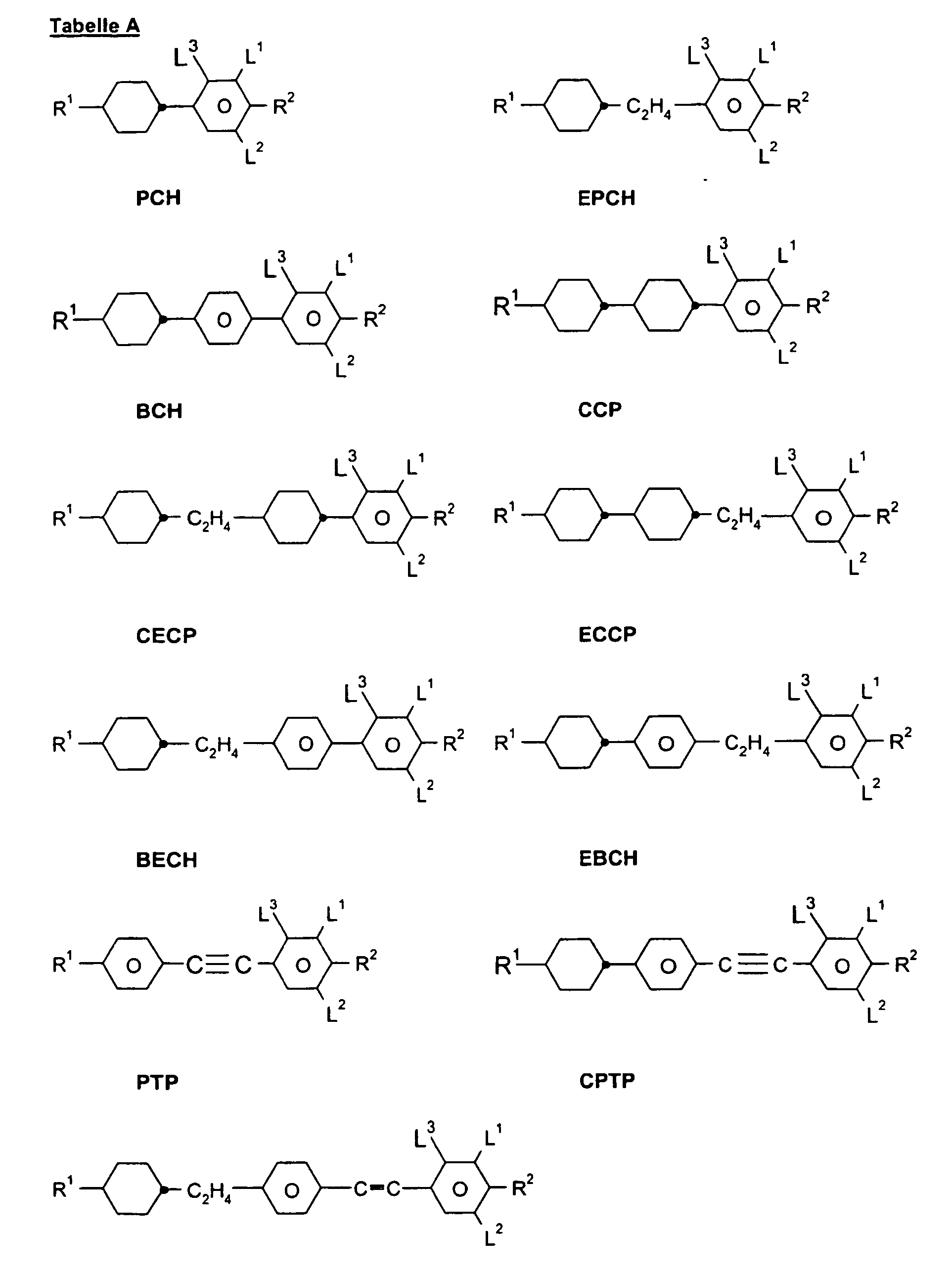

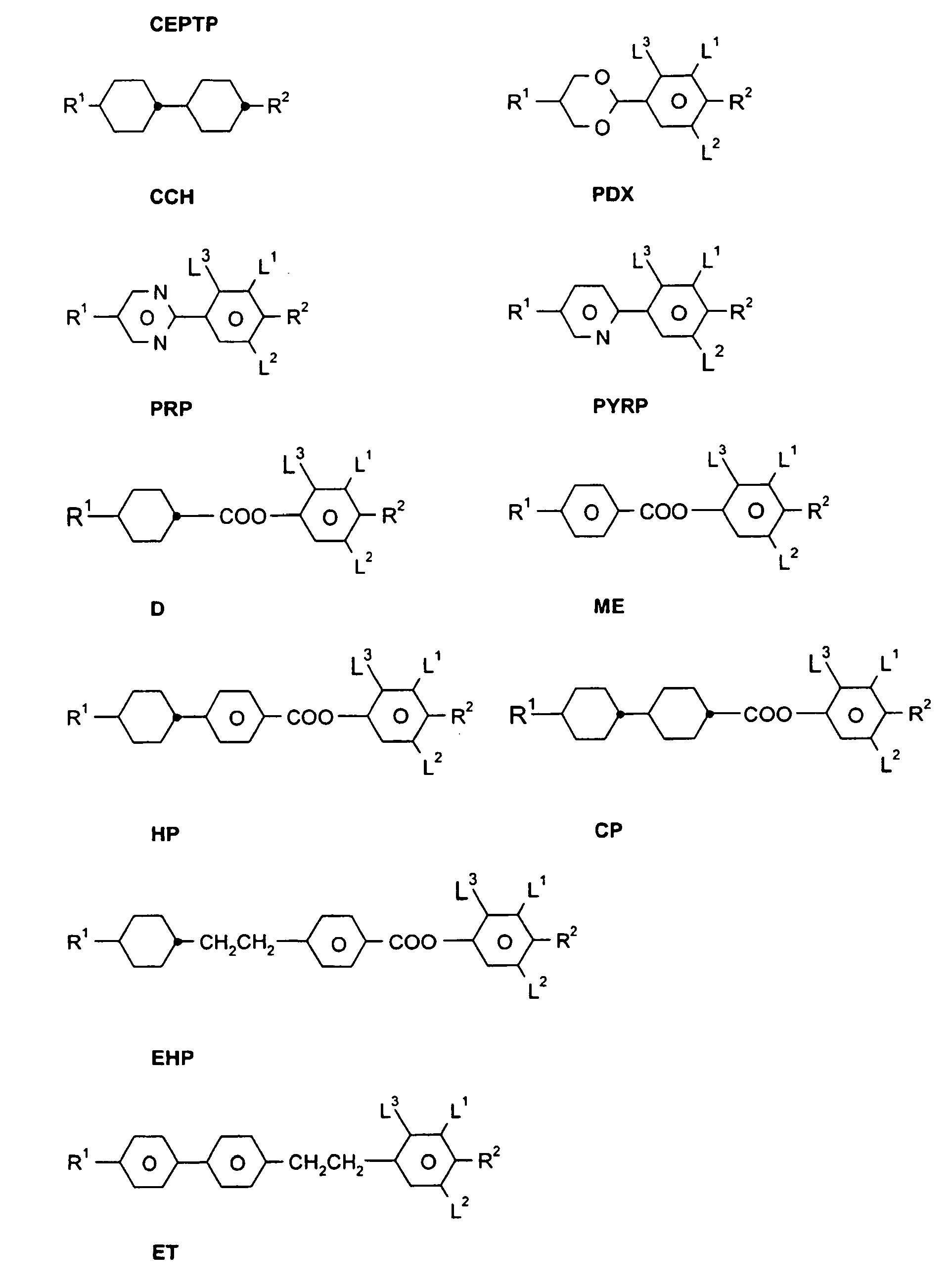

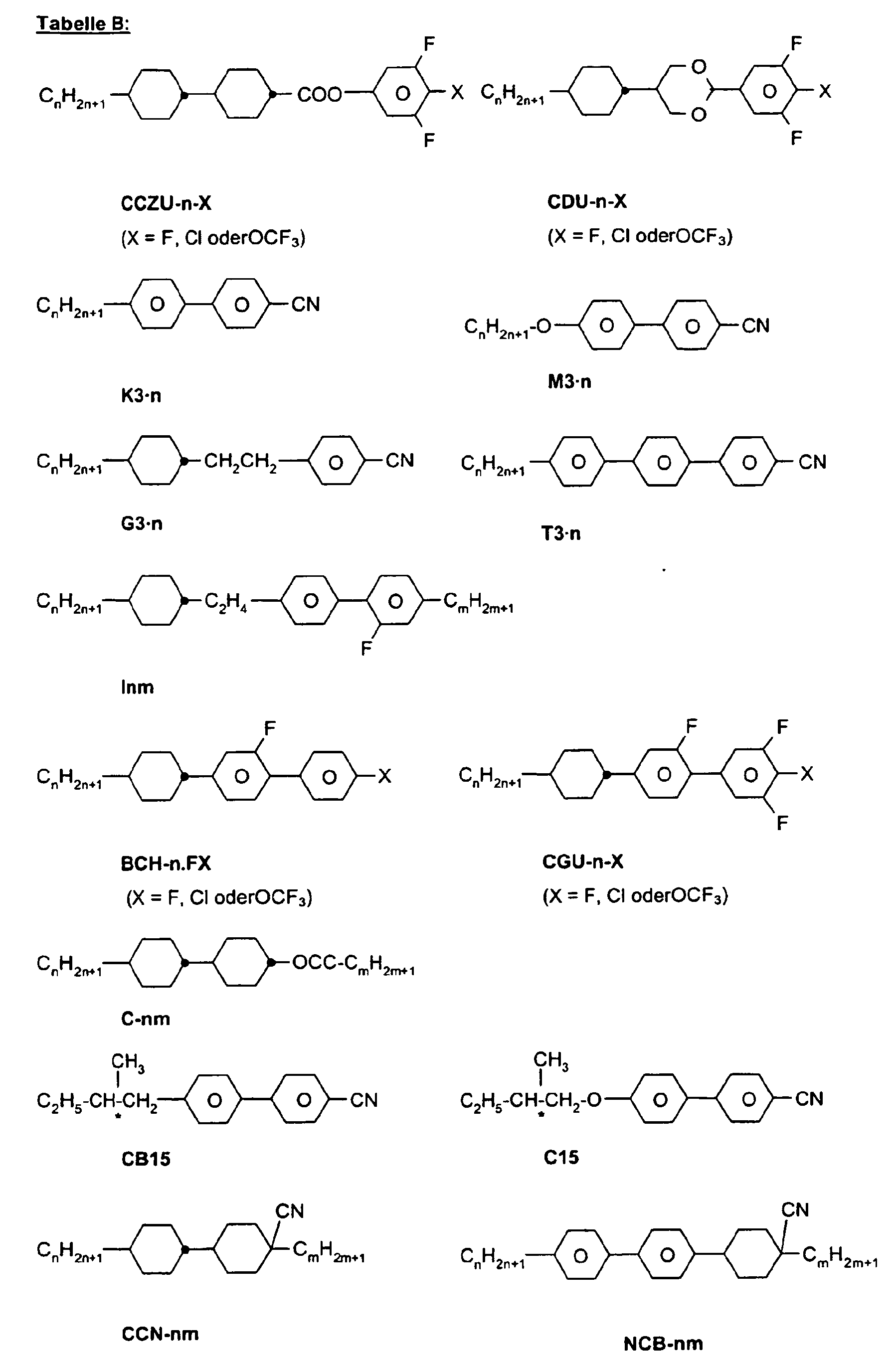

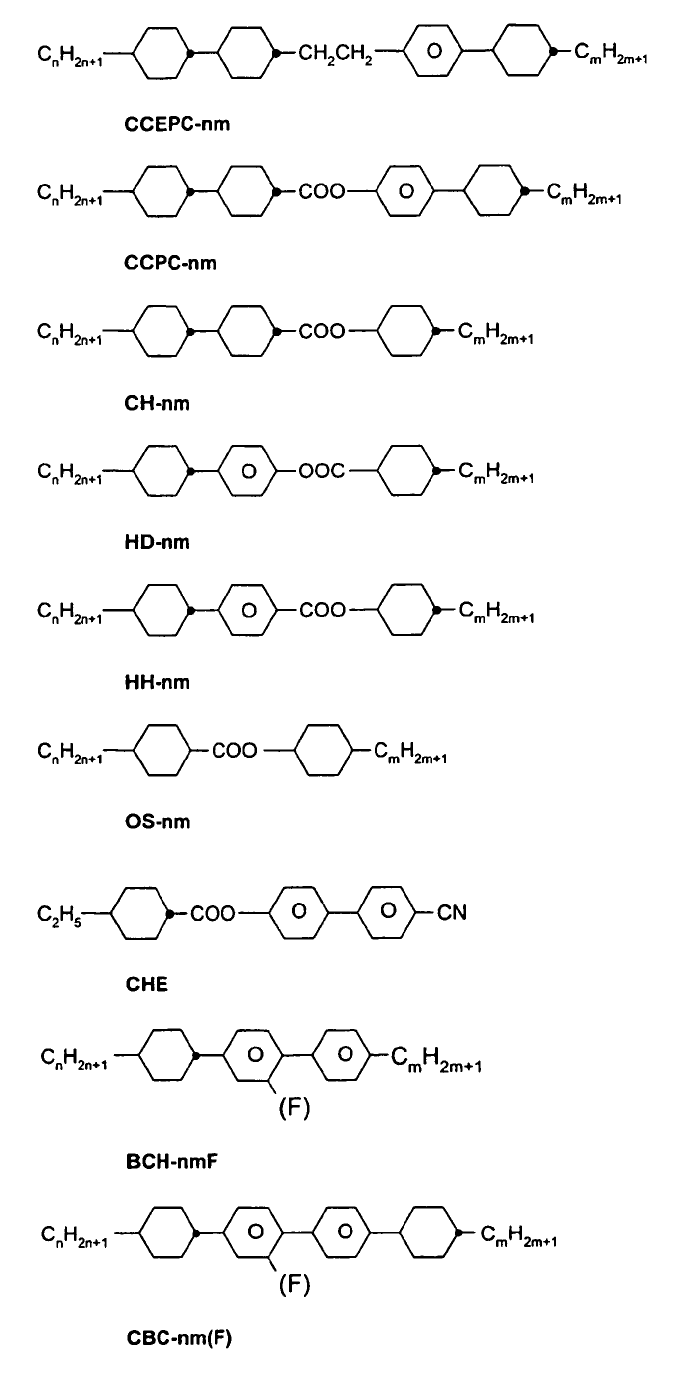

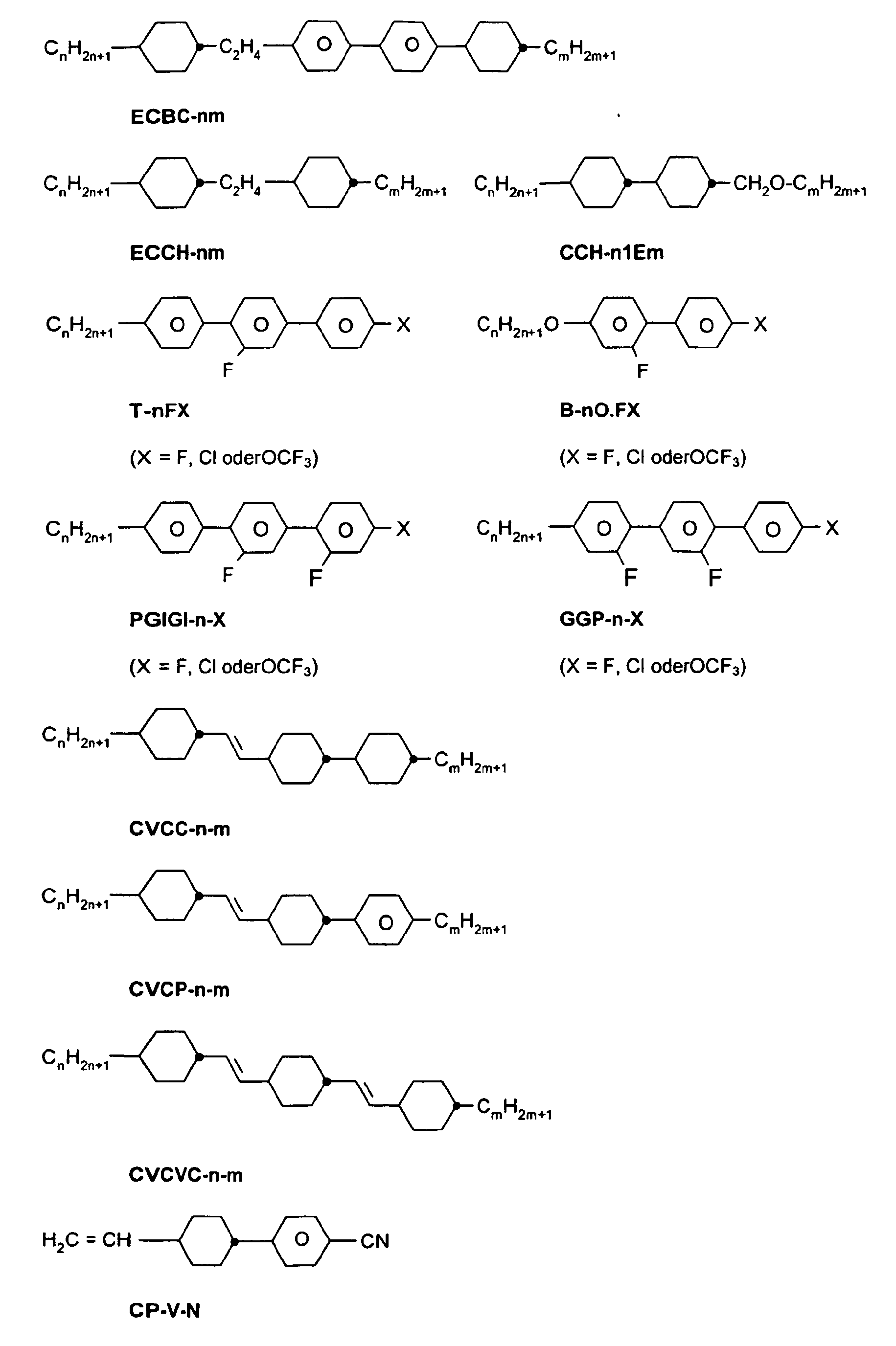

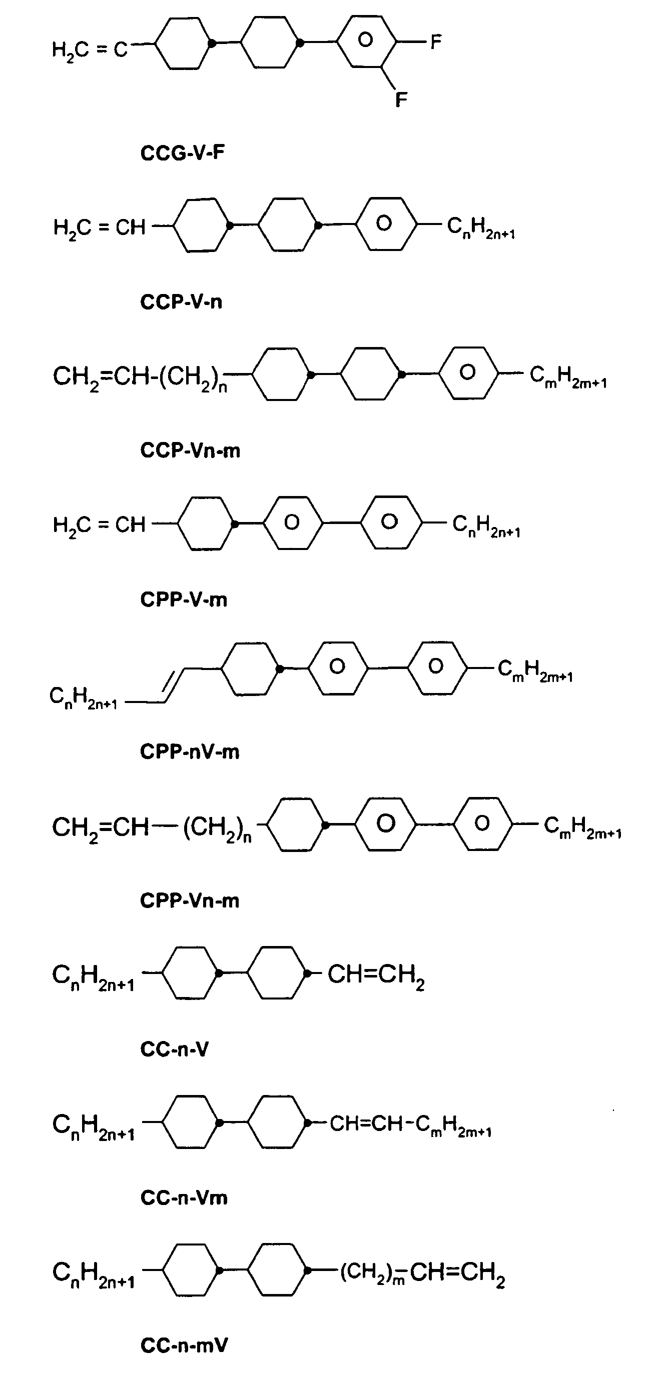

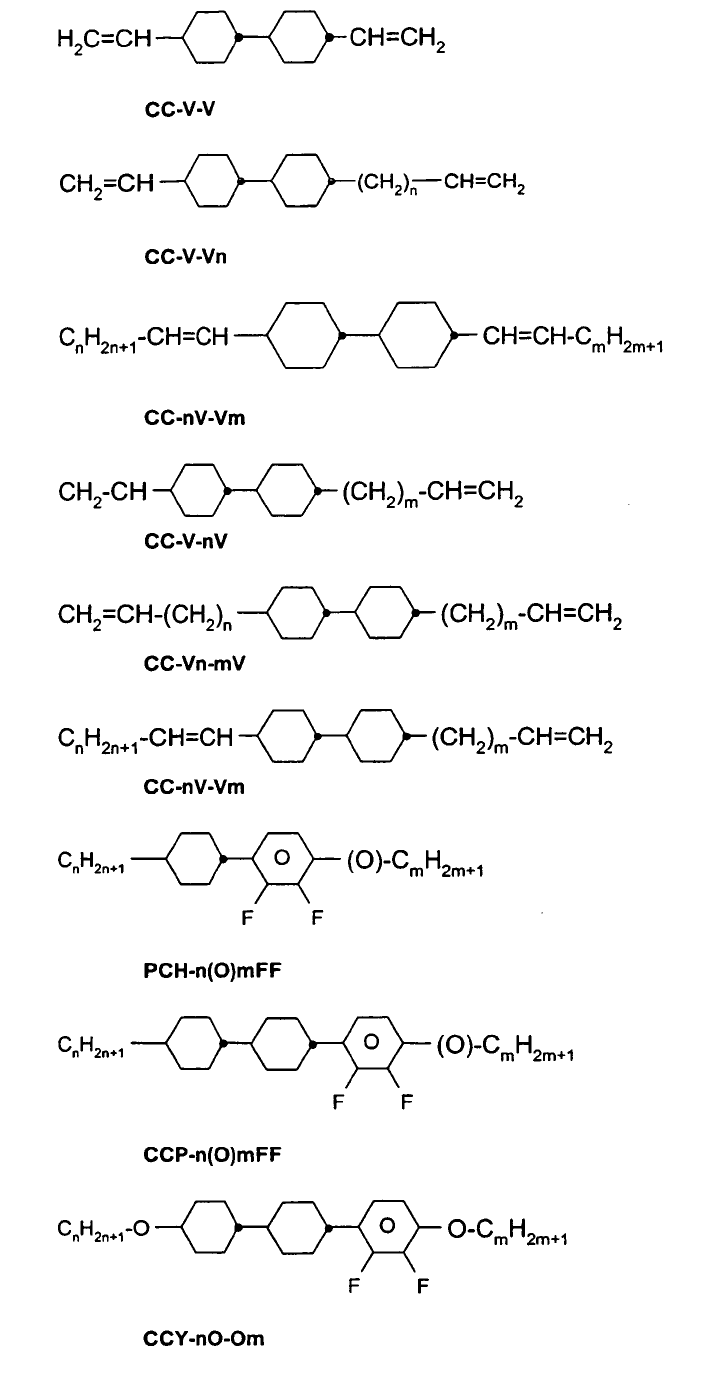

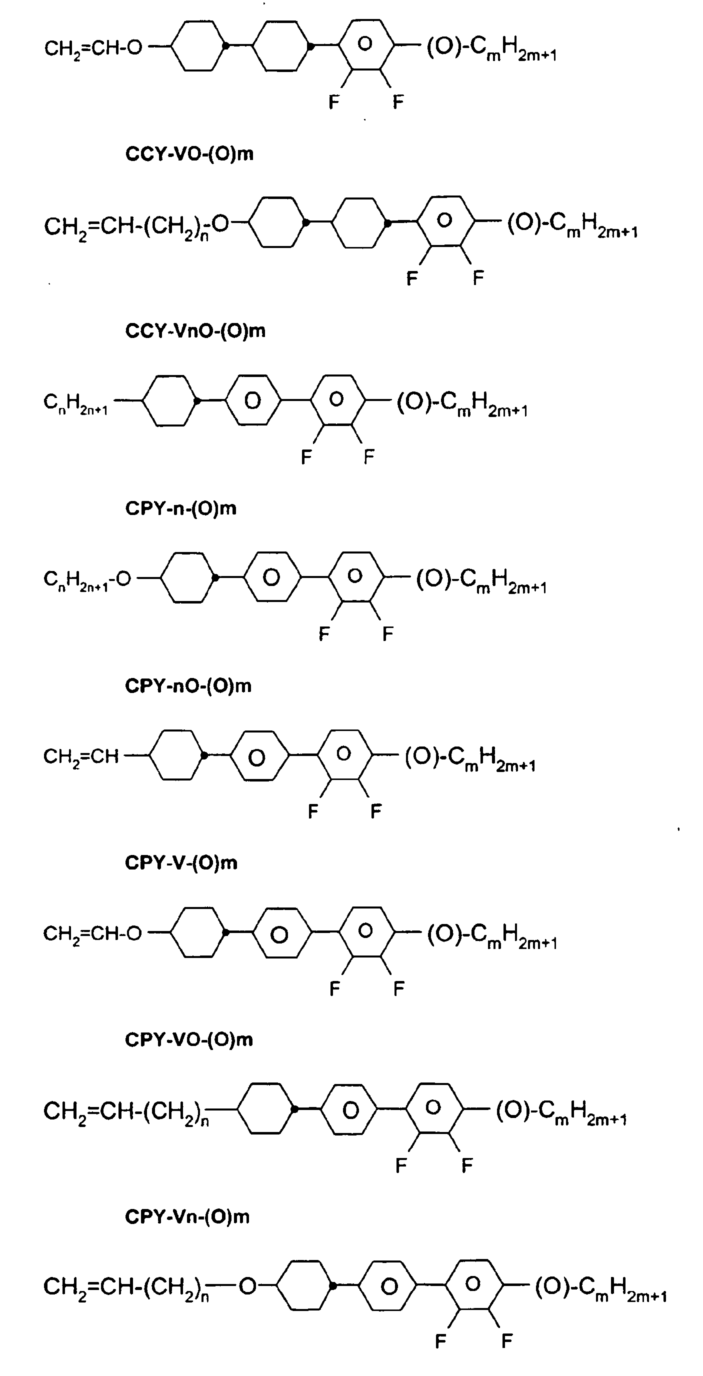

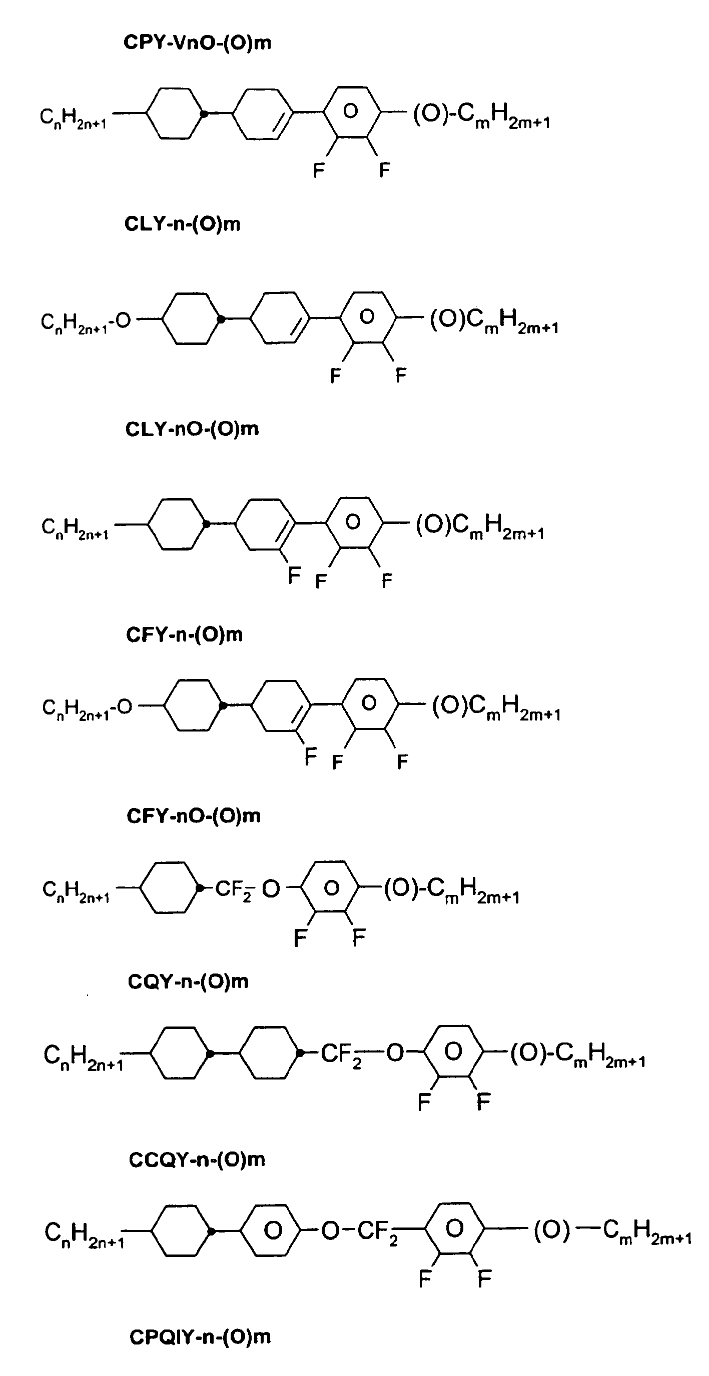

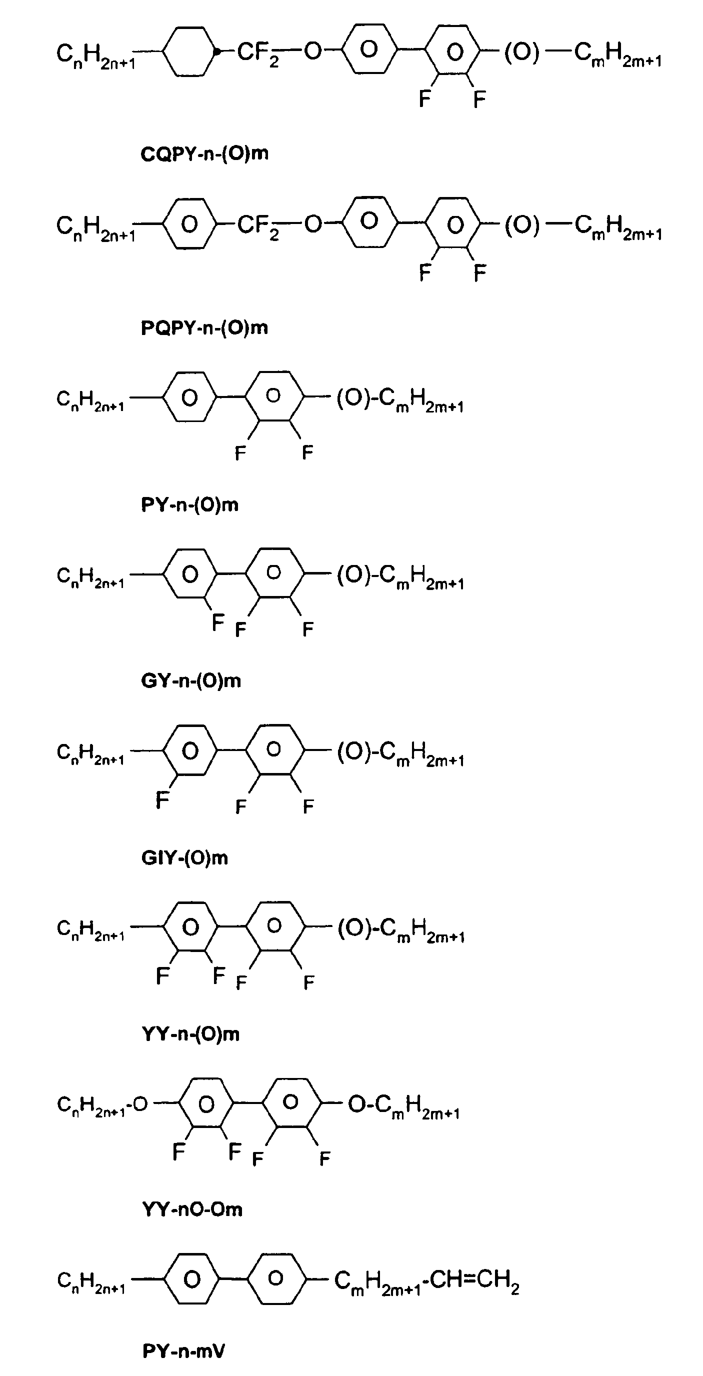

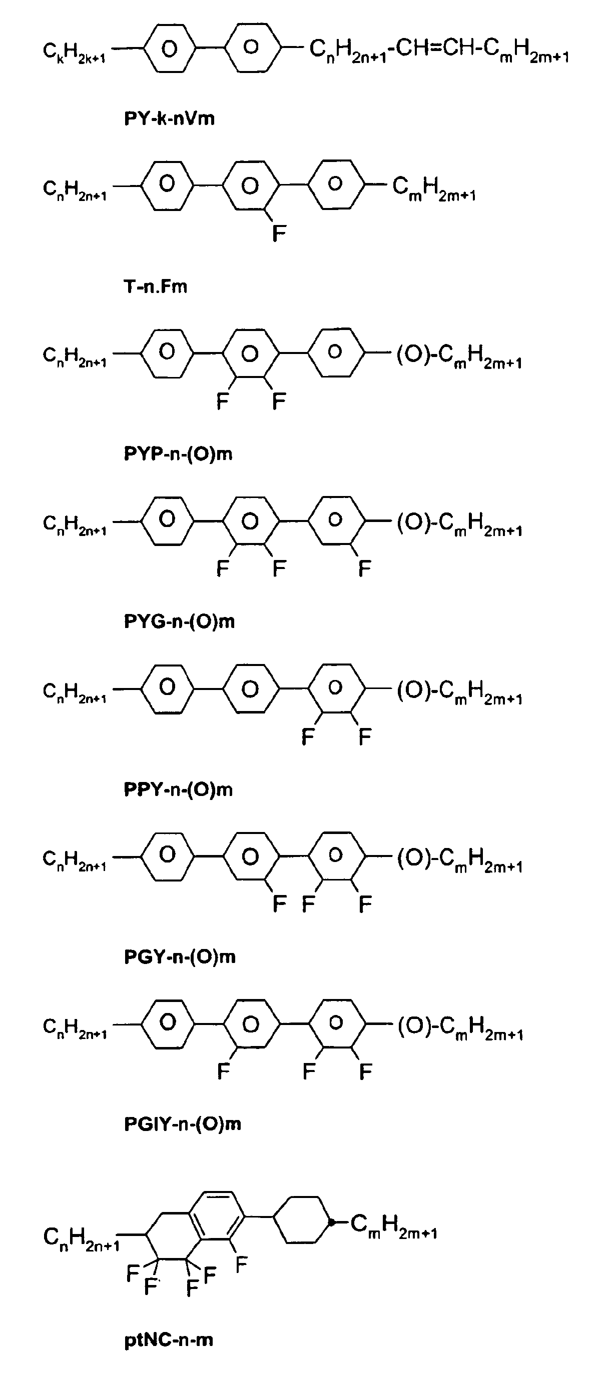

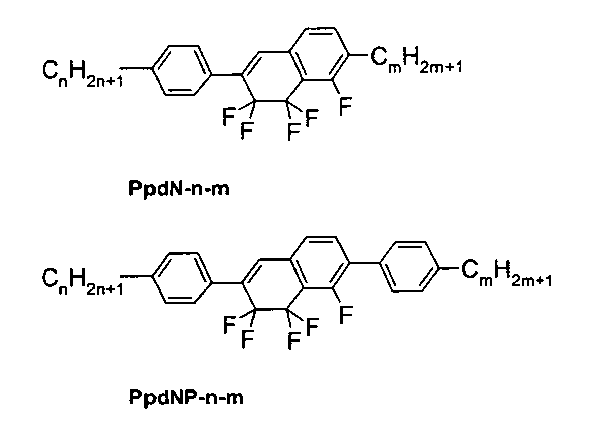

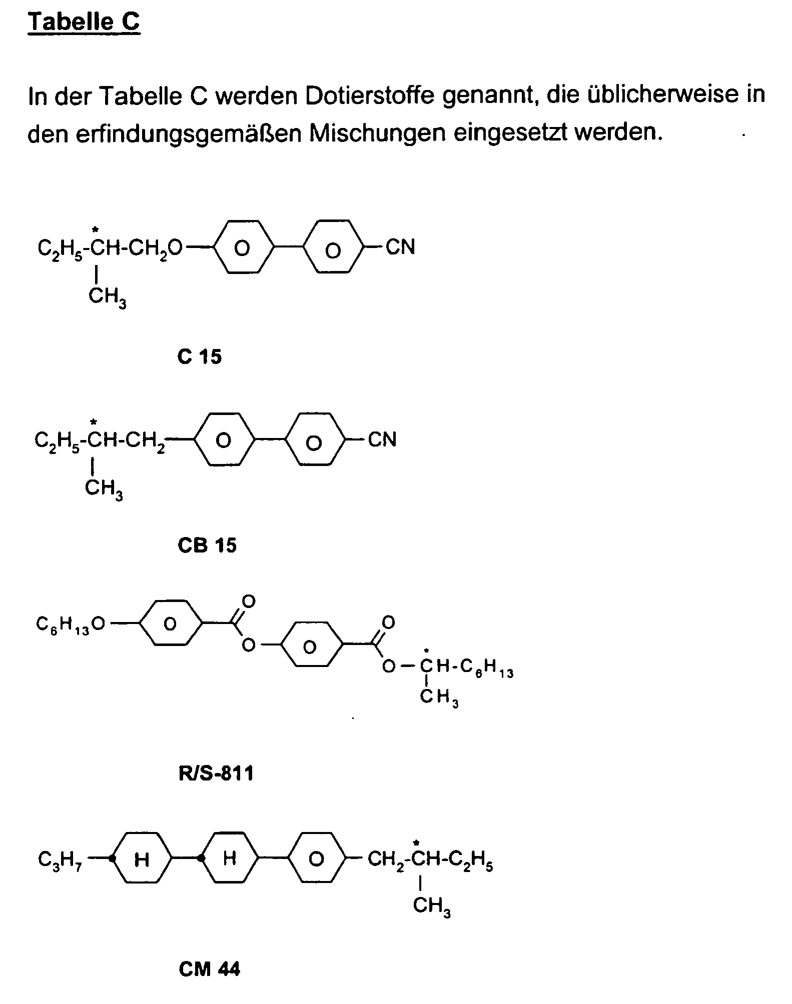

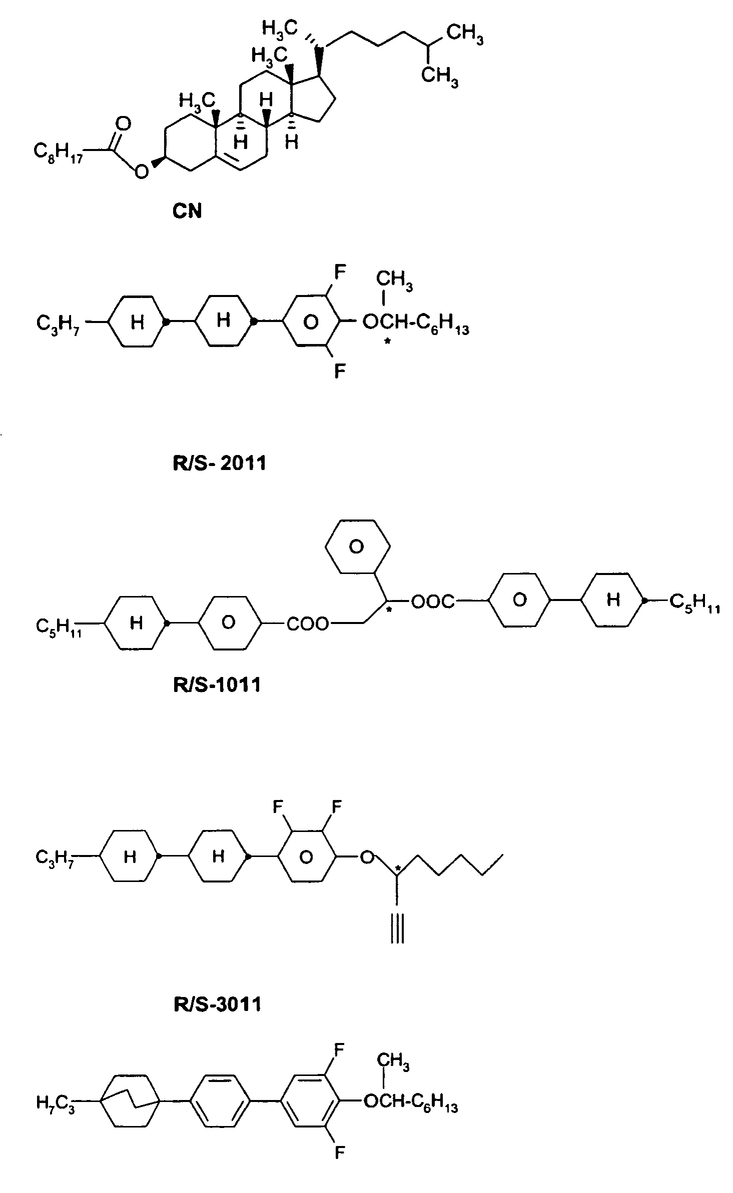

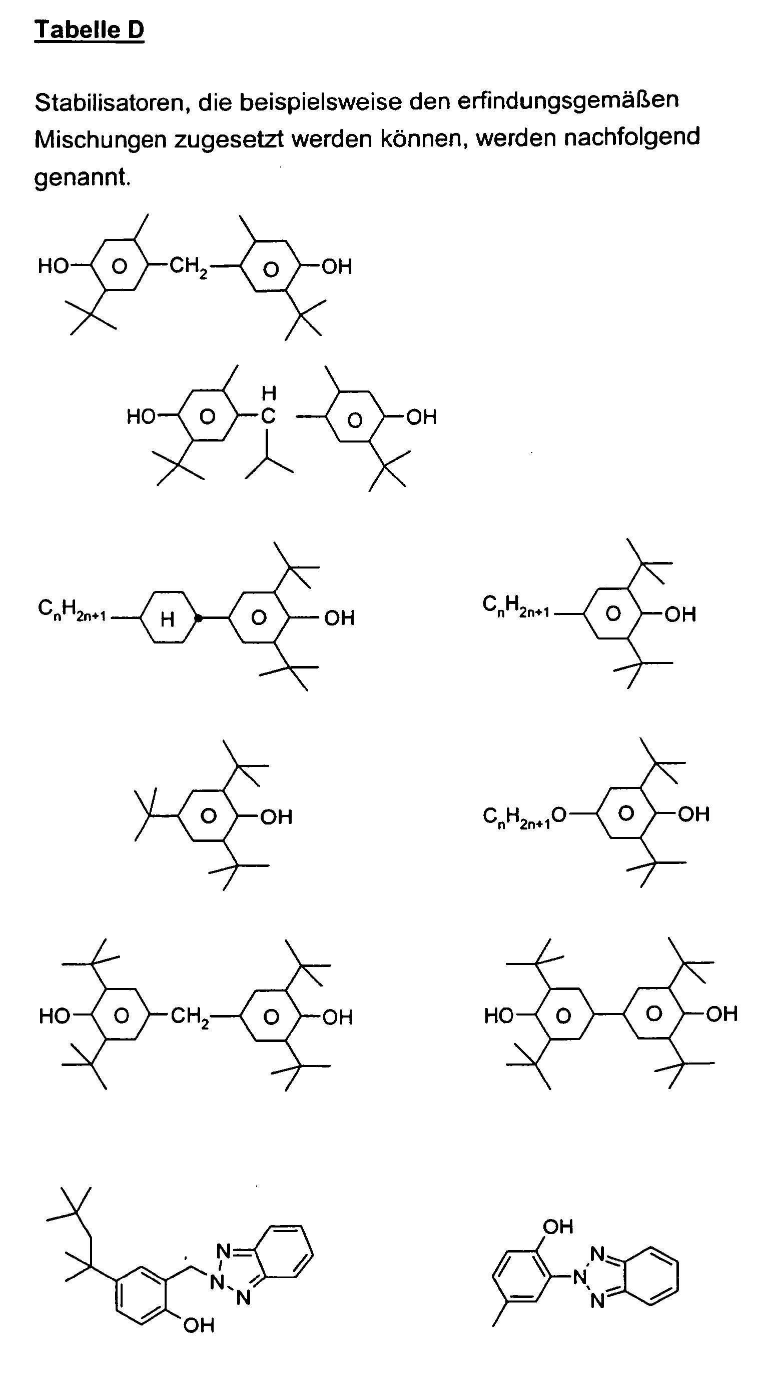

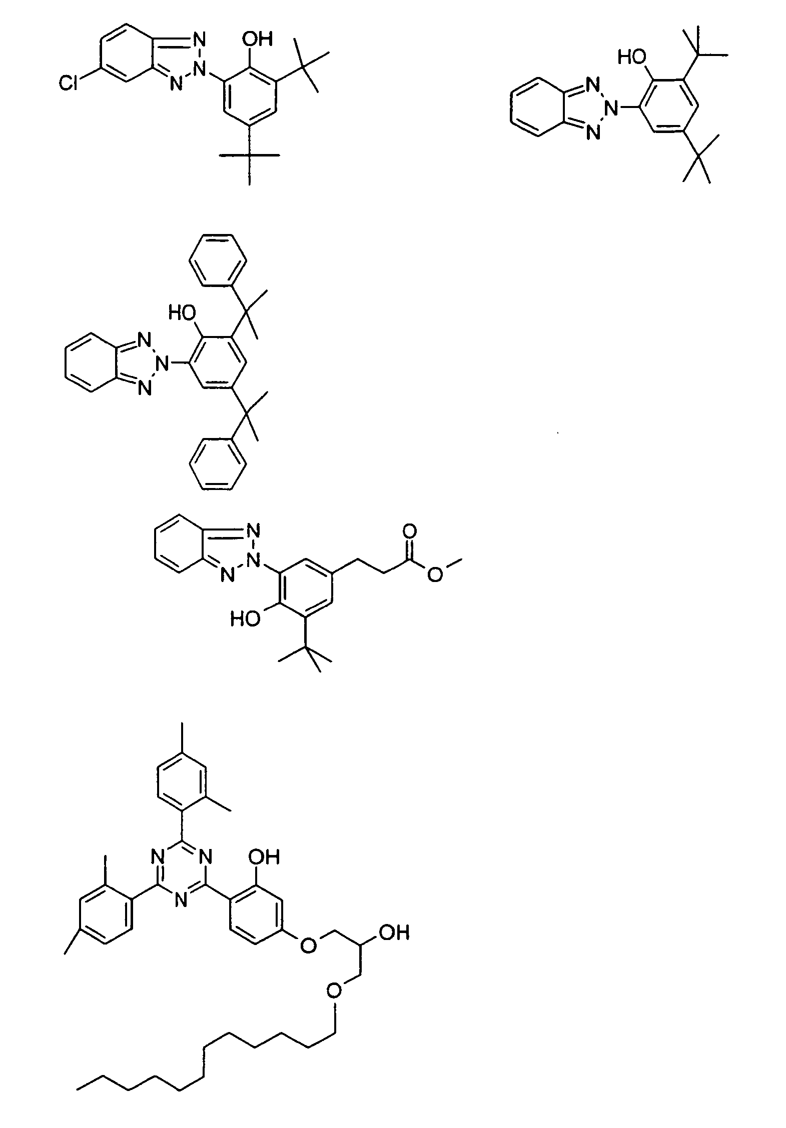

Bevorzugt enthalten die erfindungsgemäßen Flüssigkristallmedien fünf oder mehr, besonders bevorzugt sechs oder mehr und ganz besonders bevorzugt sieben oder mehr Verbindungen ausgewählt aus den Formeln der Tabellen A und B.

Bevorzugt enthalten die erfindungsgemäßen Flüssigkristallmedien zwei oder mehr, besonders bevorzugt drei oder mehr und ganz besonders bevorzugt vier oder mehr Verbindungen ausgewählt aus den Formeln der Tabelle A.

Bevorzugt enthalten die erfindungsgemäßen Flüssigkristallmedien drei oder mehr, besonders bevorzugt vier oder mehr und ganz besonders bevorzugt fünf oder mehr Verbindungen ausgewählt aus den Formeln der Tabelle B.

Diese Verbindungen sind bevorzugt Verbindungen von verschiedenen Formeln aus diesen Tabellen.

### Beispiele

Die folgenden Beispiele sollen die Erfindung erläutern, ohne sie zu begrenzen. Vor- und nachstehend bedeuten Prozentangaben Gewichtsprozent. Alle Temperaturen sind in Grad Celsius angegeben. Δn bedeutet optische Anisotropie (589 nm, 20 °C), Δε die dielektrische Anisotropie (1 kHz, 20 °C), H.R. die Voltage Holding Ratio (bei 100 °C, nach 5 Minuten im Ofen, 1 V), V₀ die Schwellenspannung, wurde bei 20 °C bestimmt.

### Beispiel 1

### 1,1,2,2,8-Pentafluor-7-(4-propylphenyl)-1,2-dihydronaphthalin [(Ib1) mit R¹=H, R²=C₃H₇]

Eine Lösung von 4,8g 1,1,2,2,8-Pentafluor-1,2-dihydronaphthalin [(Z3) mit R¹=H] in 75 ml Tetrahydrofuran wurde bei einer Temperatur unterhalb von -75°C mit 11 ml sec-Butyllithium (2M in Pentan).versetzt. Nach Zugabe von 3g Trimethylborat wurde 3 h bei gleicher Temperatur gerührt und anschließend über Nacht auf Raumtemperatur gebracht. Nach Hydrolyse mit 10%iger Salzsäure wurden 150 ml tert-Butylmethylether zugesetzt, die organische Phase abgetrennt, mit Wasser und Kochsalzlösung gewaschen und getrocknet. Die erhaltene 1,1,2,2,8-Pentafluor-1,2-dihydro-naphthalin-7-boronsäure wurde mit der äquimolaren Menge 4-Propylbrombenzol, 2 Mol-Äquivalenten Natriumcarbonat, 1 Mol-% Tetrakis(triphenylphosphin)-palladium(0) in 50 ml eines Gemisches Ethanol/Toluol/Wasser 1:2:1 (Vol.) durch eintägiges Erhitzen zum Rückfluß zur Reaktion gebracht. Nach üblicher Aufarbeitung wurde die organische Fraktion einer chromatografischen Reinigung (Kieselgel, Toluol/Heptan 2:1) unterworfen; die produkthaltigen Fraktionen wurden vereinigt, das Lösemittelgemisch im Vakuum abdestilliert und der Rückstand aus Acetonitril umkristallisiert; es wurden 1,6 g 1,1,2,2,8-Pentafluor-7-(4-propylphenyl )-1,2-dihydronaphthalin erhalten.

### Beispiel 2

### 3-Ethyl-1,1,2,2,8-pentafluor-7-(4-propylphenyl)-1,2-dihydronaphthalin [(Ib1) mit R¹=C₂H₅, R²=C₃H₇]

wurde analog Beispiel 1 erhalten.

### Beispiel 3

### 7-Ethoxy-3-(4-ethylphenyl)-1,1,2,2,8-pentafluor-1,2-dihydronaphthalin [(Ih1) mit R¹=C₂H₅, R²=C₂H₅]

wurde erhalten durch Umsetzung von 10,9 g (Z7) mit 5,3g 4-Ethylphenylboronsäure, 2 Mol-Äquivalenten Kaliumcarbonat, 1Mol-% Tetrakis(triphenylphosphin)-palladium(0) in 70 ml Ethanol/Toluol/Wasser 1:2:1 durch mehrstündiges Erhitzen zum Rückfluß, gefolgt von Standard-Aufarbeitung wie in Beispiel 1 beschrieben. Die erhaltene Verbindung [(Z8) mit q=0 und R¹=C₂H₅) wurde mittels Zinn-Granalien im Gemisch Salzsäure / Tetrahydrofuran zu (Z9) reduziert, das ohne weitere Reinigung einer Baltz-Schiemann-Reaktion zu 3-(4-Ethylphenyl)-8-fluor-2-methoxynaphthalin [(Z10) mit q=0 und R¹=C₂H₅) unterzogen wurde. Eine Lösung von 3g dieser Verbindung in 100 ml Acetonitril wurde bei Raumtemperatur mit 2 Mol-Äquivalenten F-TEDA-BF₄ versetzt; nach beendeter Reaktion wurde chromatografisch (Kieselgel, Dichlormethan) gereinigt. Das rohe 3-(4-Ethylphenyl)-1,1,8-trifluor-1 H-naphthalin-2-on [(Z11) mit q=0 und R¹=C₂H₅) wurde mit 2 Mol-Äquivalenten DAST in 50 ml Dichlormethan versetzt und bis zum Ende der Reaktion zum Rückfluß erhitzt. Nach vorsichtiger Hydrolyse wurde chromatografisch (Kieselgel, Dichlormethan) gereinigt; man erhielt 0,4 g 3-(4-Ethylphenyl)-1,1,2,2,8-pentafluor-1,2-dihydronaphthalin [(Z12) mit q=0 und R¹=C₂H₅). Dieses wurde unter den Bedingungen der ortho-Lithiierung aus Beispiel 1 in die entsprechende Boronsäure überführt, die nach Isolation aus dem Reaktionsgemisch roh mittels Wasserstoffperoxid in Diethylether in 3-(4-Ethylphenyl)-7-hydroxy-1,1,2,2,8-pentafluor-1,2-dihydro-naphthalin [(Z13 mit q=0 und R¹=C₂H₅) überführt wurde. Letztere Verbindung wurde durch mehrstündiges Erhitzen mit 1,5 Mol-Äquivalenten Ethyliodid in Gegenwart von 3 Mol-Äquivalenten Kaliumcarbonat in Aceton in 7-Ethoxy-3-(4-ethylphenyl)-1,1,2,2,8-pentafluor-1,2-dihydronaphthalin überführt, das chromatografisch (Kieselgel, Toluol) und durch Umkristallisation (Acetonitril) gereinigt wurde.

### Beispiele 4 bis 22

Analog werden hergestllt

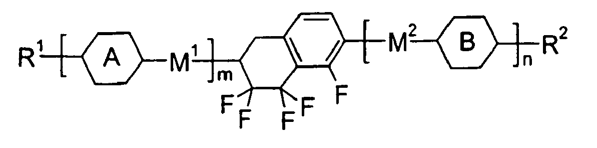

| Nr. | 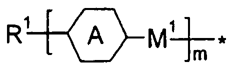 | 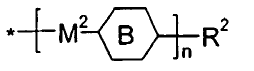 | Properties |
|---|---|---|---|
| 4 | 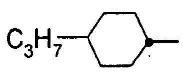 | -C₃H₇ | |
| 5 | 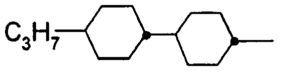 | -C₃ H₇ | |
| 6 | 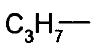 | 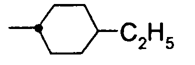 | Δε = -7,8; |
| | | | Δn = 0,074 |
| 7 | 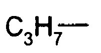 | 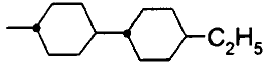 | Δε = -6,0; |
| | | | Δn = 0,074 |
| 8 | 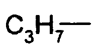 | 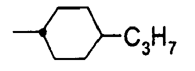 | |
| 9 | 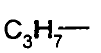 | 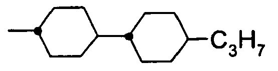 | |
| 10 | 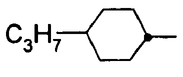 | 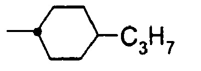 | |
| 11 | 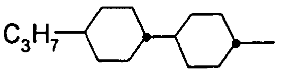 | 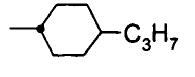 | |
| 12 | 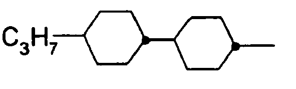 | 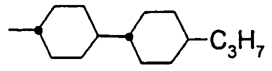 | |

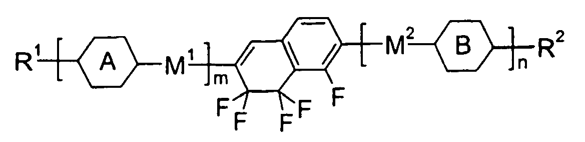

| | | | |
|---|---|---|---|
| 13 | 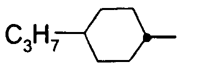 | ―CH₃ | Δε = -8;7 |
| | | | Δn = 0,107 |
| 14 | 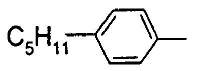 | -CH₃ | Δε = -8;8 |
| | | | Δn = 0,165 |
| 15 | H₃C- | 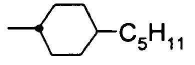 | Δε = -4,6; |
| | | | Δn = 0,106 |
| 16 | H₃C | 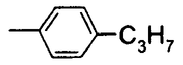 | Δε=-7,1; |
| | | | Δn=0,173 |
| 17 | H₃C- | 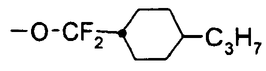 | Δε=-5,9; |
| | | | Δn=0.094 |
| 18 | H₃C- | 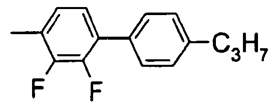 | Δε=-2,7 |
| | | | Δn=0,192 |
| 19 | H₃C- | 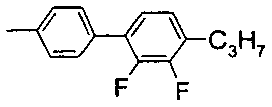 | |
| 20 | H₃C- | 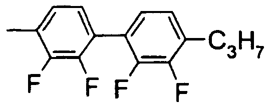 | |
| 21 | 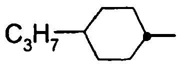 | 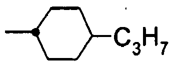 | Δε=-7,4 |
| | | | Δn=0,100 |
| 22 | C₃H₇ | 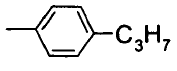 | Δε=-7,4 |
| | | | Δn = 0,209 |

### Anwendungsbeispiele

### Anwendungsbeispiel 1

Es wurde die Flüssigkristallmischung der in der folgenden Tabelle gezeigten Zusammensetzung hergestellt und untersucht. Die Ergebnisse der physikalischen Eigenschaften sind ebenfalls in der folgenden Tabelle zusammengestellt.

| Verbindung/Abkürzung | Konzentration/Massen-% | Physikalische Eigenschaften | | |
|---|---|---|---|---|
| PCH-304FF | 18,0 | T(N,l) | = 75,5 | °C |
| PCH-502FF | 9,0 | Δn (20°C, 589 nm) = | 0,0818 | |
| CCP-302FF | 8,0 | Δε (20°C, 1 kHz) | -3,7 | |
| CCP-303FF | 6,0 | γ₁ (20°C) | = 115 | mPa·s |
| CCP-402FF | 7,0 | tₛₜₒᵣₑ(-30°C) | > 1.000 | h |
| CPY-2-O2 | 9,0 | V₀(20°C) | = 2,10 | V |
| CC-5-V | 20,0 | | | |
| CC-3-V1 | 10,0 | | | |
| CCH-35 | 8,0 | | | |
| ptNC-3-3 | 5.0 | | | |
| Σ | 100,0 | | | |

Das Flüssigkristallmedium wird in eine VA-Anzeige mit TFT-Ansteuerung gefüllt. Diese Anzeige hat eine niedrige Ansteuerspannung und kurze Schaltzeiten.

### Anwendungsbeispiel 2

Es wurde die Flüssigkristallmischung der in der folgenden Tabelle gezeigten Zusammensetzung hergestellt und untersucht. Die Ergebnisse der physikalischen Eigenschaften sind ebenfalls in der folgenden Tabelle zusammengestellt.

| Verbindung/ | Konzentration/ | Physikalische Eigenschaften | | |
|---|---|---|---|---|
| Abkürzung | Massen-% | | | |
| PCH-304FF | 17,0 | T(N,l) | = 81,5 | °C |
| PCH-502FF | 12,0 | Δn (20°C, 589 nm) = | 0,1284 | |
| CPY-2-O2 | 12,0 | Δε (20°C, 1 kHz) | = -3,7 | |
| CPY-3-O2 | 12,0 | γ₁, (20°C) | = 155 | mPa·s |
| BCH-32 | 10,0 | tₛₜₒᵣₑ(-20°C) | > 1.000 | h |
| CC-5-V | 5,0 | V₀ (20 °C) | = 2,10 | V |
| CC-3-V1 | 12,0 | | | |
| CCP-V-1 | 6,0 | | | |
| CCH-35 | 6,0 | | | |
| PpdN-5-5 | 5,0 | | | |
| PpdNP-3-3 | 2,0 | | | |
| Σ | 100,0 | | | |

Das Flüssigkristallmedium wird in eine VA-Anzeige mit TFT-Ansteuerung gefüllt. Diese Anzeige hat eine niedrige Ansteuerspannung und kurze Schaltzeiten.

## Patentansprüche

1. Verbindungen der Formel (I) 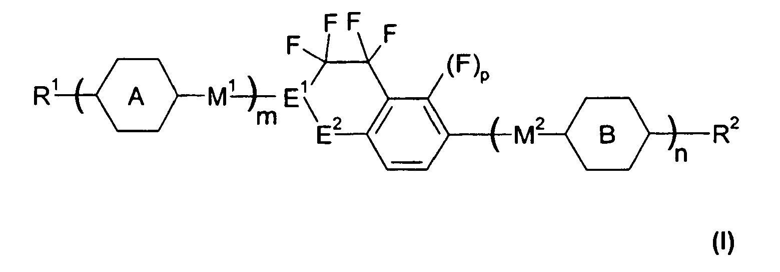 worin
**R**^{**1**} und **R**², unabhängig voneinander, H, einen linearen oder verzweigten Alkylrest mit 1 bis 12 C-Atomen oder einen linearen oder verzweigten Alkenylrest mit 2 bis 8 C-Atomen, worin auch
a) eine nicht terminale -CH₂-Gruppe durch -O- oder -C(=O)O-ersetzt sein kann und /oder
b) eine -CH₂-Gruppe durch -C≡C-, Cyclopropan-1,2-diyl oder Cyclobutan-1,3-diyl ersetzt sein kann und/oder
c) ein oder mehrere H-Atome durch F ersetzt sein können
mit der Maßgabe, dass **R**^{**1**}**, R**^{**2**} nicht zugleich H bedeuten,
**M**^{**1**} und **M**^{**2**} unabhängig voneinander -C(=O)O-, -OC(=O)-, -CH₂O-, -OCH₂-, -C≡C- , -CH₂CH₂- , -(CH₂)₄-, -CF₂CF₂-, -CF₂O- , -OCF₂- oder eine Einfachbindung.
**>E**^{**1**}**-E**^{**2**}**-** die Gruppierung >C=CH- oder >CH-CH₂-,
**m** und **n** unabhängig voneinander Null, 1 oder 2 mit der Maßgabe, dass die Summe m + n Null, 1 oder 2 beträgt,
**p** Null oder 1 und 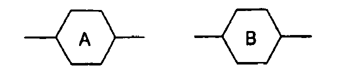
unabhängig voneinander Phenylen-1,4-diyl, gegebenenfalls ein- oder mehrfach durch F substituiert, Cyclohexan-1,4-diyl, gegebenenfalls ein- oder zweifach durch F substituiert, 1-Cyclohexen-1,4-diyl, gegebenenfalls einfach durch F substituiert oder 1,3-Dioxan-2,5-diyl bedeuten.

2. Verbindungen der Formel I nach Anspruch 1, **dadurch gekennzeichnet, dass** die Summe m + n Null oder 1 beträgt.

3. Verbindung der Formel I nach mindestens einem der Ansprüche 1 und 2, **dadurch gekennzeichnet, dass**
**R**^{**1**} und **R**^{**2**}**,** unabhängig voneinander, H, einen linearen Alkylrest mit 1 bis 12 C-Atomen oder einen linearen Alkenylrest mit 2 bis 8 C-Atomen, worin auch
a) eine nicht terminale -CH₂-Gruppe durch -O- oder -C(=O)O-ersetzt sein kann und /oder
b) eine -CH₂-Gruppe durch -C≡C-, Cyclopropan-1,2-diyl oder Cyclobutan-1,3-diyl ersetzt sein kann und/oder
c) ein oder mehrere H-Atome durch F ersetzt sein können
mit der Maßgabe, dass **R**^{**1**}**, R**^{**2**} nicht zugleich H bedeuten, bedeuten.

4. Verbindung der Formel I nach mindestens einem der Ansprüche 1 bis 3, **dadurch gekennzeichnet, dass**
**R**^{**1**}und **R**^{**2**}**,** unabhängig voneinander, H, einen linearen Alkylrest mit 1 bis 5 C-Atomen oder einen linearen Alkenylrest mit 2 bis 5 C-Atomen, worin auch
a) eine nicht terminale -CH₂-Gruppe durch -O- ersetzt sein kann und/oder
b) ein oder mehrere H-Atome durch F ersetzt sein können
mit der Maßgabe, dass **R**^{**1**}**, R**^{**2**} nicht zugleich H bedeuten, bedeuten.

5. Verbindung der Formel nach mindestens einem der Ansprüche 1 bis 4, **dadurch gekennzeichnet, dass**
**>E**^{**1**}**-E**^{**2**}**-** die Gruppierung >CH-CH₂-,
bedeutet.

6. Verbindung der Formel I nach mindestens einem der Ansprüche 1 bis 4, **dadurch gekennzeichnet, dass**
**>E**^{**1**}**-E**^{**2**}**-** die Gruppierung >C=CH-
bedeutet.

7. Verwendung von Verbindungen der Formel I gemäß mindestens einem der Ansprüche 1 bis 6 in Flüssigkristallmedien.

8. Flüssigkristallmedium enthaltend eine oder mehrere Verbindungen der Formel I nach mindestens einem der Ansprüche 1 bis 6.

9. Flüssigkristallmedium nach Anspruch 8 , **dadurch gekennzeichnet, dass** es nematisch oder cholesterisch ist und/oder dass es die Verbindung(en) der Formel I gemäß einem der Ansprüche 1 bis 6 in einem Massenanteil von 1 bis 80 Gew.-%, bezogen auf die gesamte Mischung enthält.

10. Flüssigkristalldisplay, enthaltend ein Flüssigkristallmedium nach mindestens einem der Ansprüche 8 oder 9.

## Claims

1. Compounds of the formula (I) 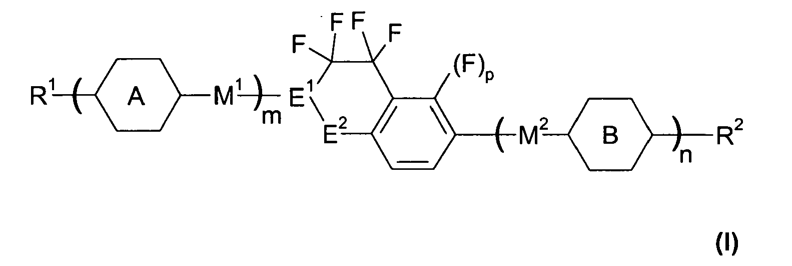 in which
**R**^{**1**} and **R**^{**2**}**,** independently of one another, denote H, a linear or branched alkyl radical having 1 to 12 C atoms or a linear or branched alkenyl radical having 2 to 8 C atoms, in which, in addition,
a) a non-terminal -CH₂- group may be replaced by -O- or -C(=O)O- and/or
b) a -CH₂- group may be replaced by -C≡C-, cyclopropane-1,2-diyl or cyclobutane-1,3-diyl and/or
c) one or more H atoms may be replaced by F,
with the proviso that R¹, R² do not simultaneously denote H,
**M**^{**1**} and **M**^{**2**}**,** independently of one another, denote -C(=O)O-, -OC(=O)-, -CH₂O-, -OCH₂-, -C≡C-, -CH₂CH₂-, -(CH₂)₄-, -CF₂CF₂-, -CF₂O-, -OCF₂- or a single bond,
**>E**¹**-E**²- denotes the >C=CH- or >CH-CH₂- group,
**m** and **n** , independently of one another, denote zero, 1 or 2, with the proviso that the sum m + n is zero, 1 or 2,
**p** denotes zero or 1 and 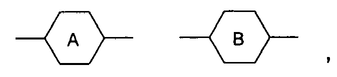
independently of one another, denote phenylene-1,4-diyl, optionally mono- or polysubstituted by F, cyclohexane-1,4-diyl, optionally mono- or disubstituted by F, 1-cyclohexene-1,4-diyl, optionally monosubstituted by F, or 1,3-dioxane-2,5-diyl.

2. Compounds of the formula I according to Claim 1, **characterised in that** the sum m + n is zero or 1.

3. Compound of the formula I according to at least one of Claims 1 and 2, **characterised in that**
**R**^{**1**} and **R**^{**2**}, independently of one another, denote H, a linear alkyl radical having 1 to 12 C atoms or a linear alkenyl radical having 2 to 8 C atoms, in which, in addition,
a) a non-terminal -CH₂- group may be replaced by -O- or -C(=O)O- and/or
b) a -CH₂- group may be replaced by -C≡C-, cyclopropane-1,2-diyl or cyclobutane-1,3-diyl and/or
c) one or more H atoms may be replaced by F,
with the proviso that **R**^{**1**}, **R**^{**2**} do not simultaneously denote H.

4. Compound of the formula I according to at least one of Claims 1 to 3, **characterised in that**
**R**^{**1**} and **R**^{**2**}, independently of one another, denote H, a linear alkyl radical having 1 to 5 C atoms or a linear alkenyl radical having 2 to 5 C atoms, in which, in addition,
a) a non-terminal -CH₂- group may be replaced by -O- and/or
b) one or more H atoms may be replaced by F,
with the proviso that **R**^{**1**}**, R**^{**2**} do not simultaneously denote H.

5. Compound of the formula I according to at least one of Claims 1 to 4, **characterised in that**
**>E**^{**1**}**-E**^{**2**}**-** denotes the >CH-CH₂- group.

6. Compound of the formula I according to at least one of Claims 1 to 4, **characterised in that**
>**E**^{**1**}**-E**^{**2**}- denotes the >C=CH- group.

7. Use of compounds of the formula I according to at least one of Claims 1 to 6 in liquid-crystal media.

8. Liquid-crystal medium comprising one or more compounds of the formula I according to at least one of Claims 1 to 6.

9. Liquid-crystal medium according to Claim 8, **characterised in that** it is nematic or cholesteric and/or **in that** it comprises the compound(s) of the formula I according to one of Claims 1 to 6 in a proportion by weight of 1 to 80% by weight, based on the mixture as a whole.

10. Liquid-crystal display containing a liquid-crystal medium according to at least one of Claims 8 or 9.

## Revendications

1. Composés de la formule (I) 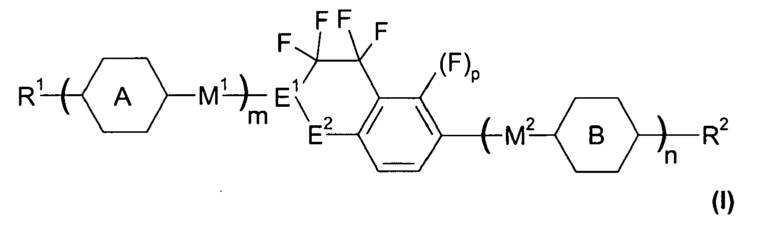 dans laquelle
**R**^{**1**}et **R**^{**2**} indépendamment l'un de l'autre, représentent H, un radical alkyle linéaire ou ramifié comportant de 1 à 12 atomes de C ou un radical alkényle linéaire ou ramifié comportant de 2 à 8 atomes de C, où, en plus,
a) un groupe -CH₂- non terminal peut être remplacé par -O-ou par -C(=O)O- et/ou
b) un groupe -CH₂- peut être remplacé par -C≡C-, cyclo-propane-1,2-diyle ou cyclobutane-1,3-diyle et/ou
c) un ou plusieurs atomes de H peuvent être remplacés par F,
étant entendu que **R**^{**1**} et **R**^{**2**} ne représentent pas de façon simultanée H,
**M**^{**1**}et **M**^{**2**} indépendamment l'un de l'autre, représentent -C(=O)O-, -OC(=O)-, -CH₂O-, -OCH₂-, -C≡C-, -CH₂CH₂-, -(CH₂)₄-, -CF₂CF₂-, -CF₂O-, -OCF₂- ou une liaison simple,
**>E**^{**1**}**-E**^{**2**}**-** représente le groupe >C=CH- ou >CH-CH₂-,
**m** et **n** indépendamment l'un de l'autre, représentent 0, 1 ou 2, étant entendu que la somme m + n vaut 0, 1 ou 2,
**p** représente 0 ou 1 ; et 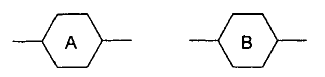
indépendamment l'un de l'autre, représentent phénylène-1,4-diyle, en option mono- ou polysubstitué par F, cyclohexane-1,4-diyle, en option mono- ou disubstitué par F, 1-cyclo-hexène-1,4-diyle, en option monosubstitué par F, ou 1,3-dioxane-2,5-diyle.

2. Composés de la formule 1 selon la revendication 1, **caractérisés en ce que** la somme m + n vaut 0 ou 1.

3. Composé de la formule I selon au moins l'une des revendications 1 et 2, **caractérisé en ce que** :
**R**^{**1**}et **R**^{**2**} indépendamment l'un de l'autre, représentent H, un radical alkyle linéaire comportant de 1 à 12 atomes de C ou un radical alkényle linéaire comportant de 2 à 8 atomes de C, où, en plus,
a) un groupe -CH₂- non terminal peut être remplacé par -O- ou par -C(=O)O- et/ou
b) un groupe -CH₂- peut être remplacé par -C≡C-, cyclopropane-1,2-diyle ou cyclobutane-1,3-diyle et/ou
c) un ou plusieurs atomes de H peuvent être remplacés par F,
étant entendu que **R**^{**1**} et **R**^{**2**} ne représentent pas de façon simultanée H.

4. Composé de la formule 1 selon au moins l'une des revendications 1 à 3, **caractérisé en ce que** :
**R**^{**1**}et **R**^{**2**} indépendamment l'un de l'autre, représentent H, un radical alkyle linéaire comportant de 1 à 5 atomes de C ou un radical alkényle linéaire comportant de 2 à 5 atomes de C, où, en plus,
a) un groupe -CH₂- non terminal peut être remplacé par -O- et/ou
b) un ou plusieurs atomes de H peuvent être remplacés par F,
étant entendu que **R**^{**1**} et **R**^{**2**} ne représentent pas de façon simultanée H.

5. Composé de la formule 1 selon au moins l'une des revendications 1 à 4, **caractérisé en ce que** :
**>E**^{**1**}**-E**^{**2**}**-** représente le groupe >CH-CH₂-.

6. Composé de la formule 1 selon au moins l'une des revendications 1 à 4, **caractérisé en ce que** :
**>E**^{**1**}**-E**^{**2**}**-** représente le groupe >C=CH-.

7. Utilisation de composés de la formule I selon au moins l'une des revendications 1 à 6 dans des milieux de cristal liquide.

8. Milieu de cristal liquide comprenant un ou plusieurs composé de la formule I selon au moins l'une des revendications 1 à 6.

9. Milieu de cristal liquide selon la revendication 8, **caractérisé en ce qu'**il est nématique ou cholostérique et/ou **en ce qu'**il comprend le ou les composés de la formule I selon l'une des revendications 1 à 6 selon une proportion en poids de 1 à 80 % en poids, sur la base du mélange pris dans sa globalité.

10. Affichage à cristaux liquides contenant un milieu de cristal liquide selon au moins l'une des revendications 8 ou 9.
